(19) Europäisches Patentamt
European Patent Office
Office européen des brevets

(11) **EP 4 778 455 A1**

(12) **EUROPEAN PATENT APPLICATION**
published in accordance with Art. 153(4) EPC

(43) Date of publication:
**22.07.2026 Bulletin 2026/30**

(21) Application number: **24865478.2**

(22) Date of filing: **11.09.2024**

(51) International Patent Classification (IPC):
**A61B 5/0536** (2021.01)

(52) Cooperative Patent Classification (CPC):
**A61B 5/0536**

(86) International application number:
**PCT/JP2024/032539**

(87) International publication number:
**WO 2025/057978 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.09.2023 JP 2023149511**

(71) Applicant: **National University Corporation Chiba
University
Chiba-shi, Chiba 263-8522 (JP)**

(72) Inventors:
• **TAKEI Masahiro
  Chiba-shi, Chiba 263-8522 (JP)**
• **DARMA Panji Nursetia
  Chiba-shi, Chiba 263-8522 (JP)**

(74) Representative: **Santarelli
Tour Trinity
1 bis Place de la Défense
92400 Courbevoie (FR)**

(54) **LOCAL BIOLOGICAL TISSUE EVALUATION DEVICE AND LOCAL BIOLOGICAL TISSUE EVALUATION METHOD**

(57)     This local biological tissue evaluation device includes a phase angle variation amount measurement unit including a sensor having a plurality of electrodes placeable on skin of a subject spaced apart from each other, and configured to inject a current or a voltage between ones of the electrodes in a state in which each of the electrodes is in contact with the skin, to measure a voltage based on a current-injection voltage-measurement pattern when the current is injected, to measure a current based on a voltage-injection current-measurement pattern when the voltage is injected between the electrodes, and to measure a feature amount obtained based on a variation amount of a phase angle for the current-injection voltage-measurement pattern or the voltage-injection current-measurement pattern, a phase angle Jacobian matrix creation unit configured to create a phase angle Jacobian matrix based on the current-injection voltage-measurement pattern or the voltage-injection current-measurement pattern, and an image reconstruction unit configured to image a phase angle distribution inside of a living body of the subject using a phase angle difference and the phase angle Jacobian matrix.

FIG. 1

**Description**

TECHNICAL FIELD

The present invention relates to a local biological tissue evaluation device and a local biological tissue evaluation method. Priority is claimed on Japanese Patent Application No. 2023-149511, filed September 14, 2023, the content of which is incorporated herein by reference.

BACKGROUND ART

It is important to evaluate the quality of local biological tissues such as muscles and internal organs for maintaining and promoting health on a daily basis. For example, the local muscle quality in each compartment of the muscle is an important element for maintaining physical function to avoid the risk of lifestyle-related diseases or to improve the quality of life.

In general, muscle quality is evaluated by three muscle quality indexes, muscle aging (MA), fatty infiltration (FI), and muscle cross-sectional area (CSA). These muscle quality indexes vary greatly depending on each muscle compartment (also referred to as a compartment, and each muscle compartment has a unique name). Therefore, clinically, it is desirable to locally evaluate the muscle quality indexes of each muscle compartment rather than evaluating the entire body globally. Furthermore, it goes without saying that a low-invasive, low-cost, and simple evaluation method is more desirable from the viewpoint of daily monitoring and the like. The evaluation of the local muscle quality indexes has been performed by immunohistochemistry, magnetic resonance imaging, dual-energy X-ray absorptiometry, ultrasound, and the like in the related art.

For example, muscle aging (MA) is evaluated invasively by immunohistochemistry in which muscle tissue is snap-frozen and then the contours of hundreds of muscle fibers are drawn using a threshold value function. Fatty infiltration (FI) is calculated from an image obtained by magnetic resonance imaging using software. The muscle cross-sectional area (CSA) is obtained by the transmission intensity of dual-energy X-ray absorptiometry.

However, these evaluation methods of the local muscle quality indexes in the related art are invasive, radioactive, or expensive, and are large-scale. Therefore, the methods are not practical for daily monitoring.

On the other hand, although it is an evaluation method of global muscle quality indexes, a bio-impedance analysis (BIA) is a low-invasive, low-cost, and simple muscle quality evaluation method. In the bio-impedance analysis (BIA), generally, four electrodes are brought into contact with the limbs of a living body, a current is injected from two of the electrodes, a voltage is measured from the other two electrodes, a phase angle ($\theta$) between the injected current (I) and the measured voltage (V) is measured, and a part of the global muscle quality indexes is evaluated. Here, the phase angle ($\theta$) is defined as $\theta = \tan^{-1}(Z''/Z')$ using a resistance (resistive) component (real part of complex impedance) Z' of impedance and a reactance component (imaginary part of complex impedance) Z'' of impedance. In the bio-impedance analysis (BIA), the global fatty infiltration (FI) and the global muscle volume (not the muscle cross-sectional area) are roughly evaluated from the global phase angle ($\theta$) of the living body, and the three muscle quality indexes of each muscle compartment are not locally evaluated.

Furthermore, as a low-invasive, low-cost, simple, and local evaluation method, Non-Patent Document 1 proposes capacitively coupled dielectric spectroscopy tomography (C4D = Capacitively Coupled Contactless Conductivity Detection) for an air-driven pneumatic powder conveying pipeline in a factory or the like, based on phase angle measurement and a phase angle Jacobian matrix (phase angle sensitivity matrix), and discloses a phase angle imaging method.

Citation List

Non-Patent Document

Non Patent Document 1: Y. Jiang and M. Soleimani, "Capacitively Coupled Phase-based Dielectric Spectroscopy Tomography" Sci. Rep., vol. 8, no. 1, p. 17526, 2018

SUMMARY OF INVENTION

Technical Problem

The method of Non Patent Document 1 assumes the evaluation of a local field in an industrial product, and is not intended for muscle quality indexes. In addition, according to the international electrotechnical standard (IEC) standard, the method is a method for injecting a high voltage of 1 V at an applied voltage frequency f = 10 kHz to 15 MHz to measure a current I, to compensate for the application of non-contact electrical capacitance tomography, and the high voltage is injected via a sensor. Furthermore, in the method of Non-Patent Document 1, the Jacobian matrix J obtained from the difference from physiological saline is used as the background electrical conductivity, but since the localized biological tissue (local biological tissue) is a frequency-dependent substance, there is an issue that it is difficult to evaluate the quality of the local biological tissue even when the method of Non-Patent Document 1 is simply applied to the living body. In the end, even when this method is applied to the living body, it is difficult to perform imaging.

The present invention has been made in view of the above circumstances, and an object of the present invention is to provide a local biological tissue evaluation device and a local biological tissue evaluation method capable of evaluating the quality of a localized biological tissue in a low-invasive, low-cost, and simple manner.

Solution to Problem

To solve the above problems, the present invention proposes the following means.

<1> A local biological tissue evaluation device according to an aspect 1 of the present invention includes a phase angle variation amount measurement unit including a sensor having a plurality of electrodes placeable on skin of a subject spaced apart from each other, and configured to inject a current or a voltage between ones of the electrodes in a state in which each of the electrodes is in contact with the skin, to measure a voltage based on a current-injection voltage-measurement pattern when the current is injected, to measure a current based on a voltage-injection current-measurement pattern when the voltage is injected between the electrodes, and to measure a feature amount obtained based on a variation amount of a phase angle for the current-injection voltage-measurement pattern or the voltage-injection current-measurement pattern, a phase angle Jacobian matrix creation unit configured to create a phase angle Jacobian matrix based on the current-injection voltage-measurement pattern or the voltage-injection current-measurement pattern, and an image reconstruction unit configured to image a phase angle distribution inside of a living body of the subject using the feature amount and the phase angle Jacobian matrix.

<2> An aspect 2 of the present invention may be the local biological tissue evaluation device according to the aspect 1, in which the phase angle Jacobian matrix creation unit creates mesh coordinates of a measurement location of the subject and an equation of a relationship of an n-th mesh in an m-th measurement pattern, and derives, as an element of the phase angle Jacobian matrix obtained as a solution of the equation, a current and an electric potential at each mesh coordinate, and a phase angle of an impedance, which is the ratio of the voltage to the current.

<3> An aspect 3 of the present invention may be the local biological tissue evaluation device according to the aspect 1 or 2,

in which the feature amount is a phase angle difference represented by Equation (1),

$$\Delta\theta_m = \theta_m(f) - \theta_m(f_0) \ ... \ \text{Equation (1)}$$

in Equation (1),
$\Delta\theta_m$ is a phase angle difference in the m-th current-injection voltage-measurement pattern or in the m-th voltage-injection current-measurement pattern,
$\theta_m(f)$ is a phase angle at a high frequency f in the m-th current-injection voltage-measurement pattern or in the m-th voltage-injection current-measurement pattern, and
$\theta_m(f_0)$ is a phase angle at a low frequency $f_0$ in the m-th current-injection voltage-measurement pattern or in the m-th voltage-injection current-measurement pattern.

<4> An aspect 4 of the present invention may be the local biological tissue evaluation device according to any one of the aspects 1 to 3,
further including: a local biological tissue evaluation unit configured to evaluate a local biological tissue using the imaged phase angle distribution.

<5> An aspect 5 of the present invention may be the local biological tissue evaluation device according to any one of the aspects 1 to 4,
in which the local biological tissue evaluation unit includes a local biological tissue aging evaluation unit configured to calculate a spatial mean of the phase angle distribution from the phase angle distribution.

<6> An aspect 6 of the present invention may be the local biological tissue evaluation device according to any one of the aspects 1 to 5,
in which the local biological tissue evaluation unit includes a local biological tissue fatty infiltration evaluation unit configured to create a local biological tissue phase angle distribution, which is a phase angle distribution of a local biological tissue, by assigning the phase angle distribution to each local biological tissue of the subject.

<7> An aspect 7 of the present invention may be the local biological tissue evaluation device according to the aspect 6, in which the local biological tissue fatty infiltration evaluation unit calculates a spatial mean of the local biological tissue phase angle distribution from the local biological tissue phase angle distribution.

<8> An aspect 8 of the present invention may be the local biological tissue evaluation device according to the aspect 6 or 7,

in which the local biological tissue evaluation unit further includes a local biological tissue cross-sectional area evaluation unit configured to calculate a local biological tissue cross-sectional area ratio after performing binarization processing on the local biological tissue phase angle distribution.

<9> A local biological tissue evaluation method according to a ninth aspect of the present invention includes a first step of using a sensor including a plurality of electrodes placeable on skin of a subject spaced apart from each other, injecting a current or a voltage between the electrodes in a state in which each of the electrodes is in contact with the skin, measuring a voltage based on a current-injection voltage-measurement pattern when the current is injected, measuring a current based on a voltage-injection current-measurement pattern when the voltage is injected between the electrodes, and measuring a feature amount obtained based on a variation amount of a phase angle for the current-injection voltage-measurement pattern or the voltage-injection current-measurement pattern; a second step of creating a phase angle Jacobian matrix based on the current-injection voltage-measurement pattern or the voltage-injection current-measurement pattern; and a third step of imaging a phase angle distribution inside of a living body of the subject from the phase angle difference and the phase angle Jacobian matrix.

<10> An aspect 10 of the present invention may be the local biological tissue evaluation method according to the aspect 9,

in which in the second step, mesh coordinates of a measurement location of the subject and an equation of a relationship of an n-th mesh in an m-th measurement pattern are created, and a current and an electric potential at each mesh coordinate, and a phase angle of an impedance, which is a ratio of the voltage to the current, are derived as an element of the phase angle Jacobian matrix obtained as a solution of the equation.

<11> An aspect 11 of the present invention may be the local biological tissue evaluation method according to the aspect 9 or 10,

in which the feature amount is a phase angle difference represented by Equation (2),

$$\Delta\theta_m = \theta_m(f) - \theta_m(f_0) \ldots \text{Equation (2)}$$

in Equation (2),

$\Delta\theta_m$ is a phase angle difference in the m-th current-injection voltage-measurement pattern or in the m-th voltage-injection current-measurement pattern,

$\theta_m(f)$ is a phase angle at a high frequency f in the m-th current-injection voltage-measurement pattern or in the m-th voltage-injection current-measurement pattern, and

$\theta_m(f_0)$ is a phase angle at a low frequency $f_0$ in the m-th current-injection voltage-measurement pattern or in the m-th voltage-injection current-measurement pattern.

<12> An aspect 12 of the present invention may be the local biological tissue evaluation method according to any one of the aspects 9 to 11,

further including: a fourth step of evaluating a local biological tissue using the imaged phase angle distribution.

<13> An aspect 13 of the present invention may be the local biological tissue evaluation method according to the aspect 12,

in which in the fourth step, a spatial mean of the phase angle distribution is calculated from the phase angle distribution.

<14> An aspect 14 of the present invention may be the local biological tissue evaluation method according to the aspect 12 or 13,

in which in the fourth step, the phase angle distribution is assigned to each local biological tissue of the subject to create a local biological tissue phase angle distribution, which is a phase angle distribution of the local biological tissue.

<15> An aspect 15 of the present invention may be the local biological tissue evaluation method according to the aspect 14,

in which in the fourth step, after binarization processing is performed on the local biological tissue phase angle distribution, a local biological tissue cross-sectional area ratio is calculated.

Advantageous Effects of Invention

According to the above-described aspects of the present invention, each of the above-described aspects of the present invention can provide a local biological tissue evaluation device and a local biological tissue evaluation method capable of evaluating the quality of a localized biological tissue in a low-invasive, low-cost, and simple manner.

BRIEF DESCRIPTION OF DRAWINGS

[FIG. 1] A functional block diagram of a local biological tissue evaluation device according to an embodiment of the present invention.
[FIG. 2] A schematic diagram showing a phase angle variation amount measurement unit shown in FIG. 1, a method for measuring a phase angle difference by the phase angle variation amount measurement unit, and a control unit.
[FIG. 3] A flowchart showing a flow of measuring a measured phase angle difference $\Delta\theta_m$ based on a current-injection voltage-measurement pattern.
[FIG. 4] A flowchart for creating $^{\Phi}J$ in a phase angle Jacobian matrix creation unit.
[FIG. 5] A diagram showing an example of (a) an electrode arrangement on a calf and a mesh in accordance with the flow shown in FIG. 4, (b) a diagram classifying cross-sectional positions of elements in the calf, (c) a current-injection voltage-measurement pattern, (d) a created resistance Jacobian matrix, (e) a created reactance Jacobian matrix, and (f) a created phase angle Jacobian matrix $^{\Phi}J$ (f).
[FIG. 6] A diagram showing an example of an image of a phase angle distribution $\Phi_p$ in a p-th section and an image of a binarized phase angle distribution $\Phi_p{}^\wedge$.
[FIG. 7] A flowchart of a local biological tissue evaluation method according to an embodiment of the present invention.
[FIG. 8] A configuration of a muscle used in a simulation of muscle quality evaluation.
[FIG. 9] A diagram showing a calculated phase angle distribution image $\Phi$.
[FIG. 10] A diagram showing a result of a spatial mean phase angle $<\Phi>$ of each muscle compartment.
[FIG. 11] A binarized phase angle distribution image.
[FIG. 12] A diagram showing a local muscle cross-sectional area ratio of each muscle compartment.
[FIG. 13] A schematic diagram of a device used in Experimental Example 2.
[FIG. 14] A calculated phase angle distribution image.
[FIG. 15] A diagram showing a spatial mean phase angle $<\Phi>$ of each subject.
[FIG. 16] An ultrasound image of each subject for comparison.
[FIG. 17] A diagram showing a spatial mean phase angle $<\Phi_{p1}>$ of a first muscle compartment, a spatial mean phase angle $<\Phi_{p2}>$ of a second muscle compartment, a spatial mean phase angle $<\Phi_{p3}>$ of a third muscle compartment, a spatial mean phase angle $<\Phi_{p4}>$ of a fourth muscle compartment, and a local fatty infiltration uFI obtained from an ultrasound image of each subject.
[FIG. 18] A binarized phase angle image of each subject.

DESCRIPTION OF EMBODIMENTS

Hereinafter, a local biological tissue evaluation device 100 according to an embodiment of the present invention will be described with reference to the drawings. Hereinafter, a muscle will be described as an example of a biological tissue. However, the biological tissue is not limited to the muscle. Examples of the biological tissue include a heart, lungs, a liver, kidneys, a spleen, a pancreas, a stomach, a small intestine, a large intestine, a bladder, and a gallbladder. In the following description, the muscle includes three types of a skeletal muscle that moves the body, a smooth muscle that is present in a vascular wall, and a myocardium that constructs the heart, but the skeletal muscle may be referred to as the muscle as a representative term.

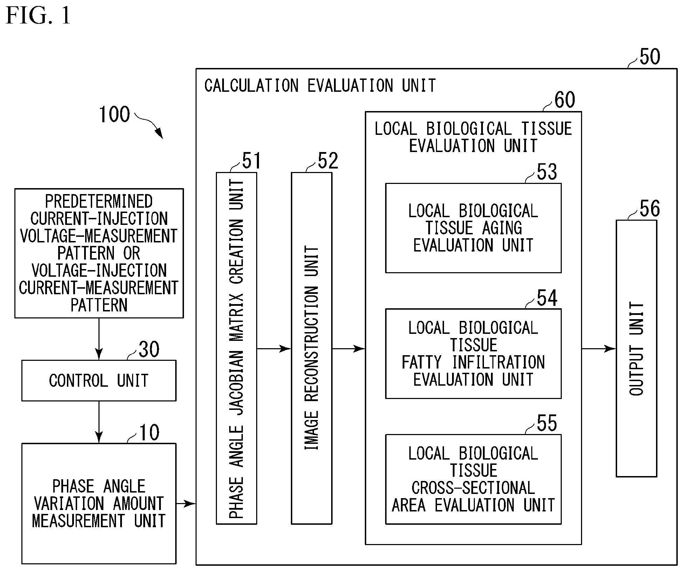

As shown in FIG. 1, the local biological tissue evaluation device 100 includes a control unit 30, a phase angle variation amount measurement unit 10, and a calculation evaluation unit 50. The calculation evaluation unit 50 includes a phase angle Jacobian matrix creation unit 51, an image reconstruction unit 52, and an output unit 56. The local biological tissue evaluation device 100 may further include a local biological tissue evaluation unit 60. The local biological tissue evaluation unit 60 includes a local biological tissue aging evaluation unit 53, a local biological tissue fatty infiltration evaluation unit 54, and a local biological tissue cross-sectional area evaluation unit 55. The calculation evaluation unit 50 of the local biological tissue evaluation device 100 includes, for example, a central processing unit (CPU), a read only memory (ROM), a random access memory (RAM), and a hard disk drive (HDD)/solid state drive (SSD). The phase angle Jacobian matrix creation unit 51, the image reconstruction unit 52, the local biological tissue evaluation unit 60, and the output unit 56 are realized by executing a predetermined program on the CPU. The program may be acquired via a recording medium or may be acquired via a network. In addition, a dedicated hardware configuration for realizing the configuration of the local biological tissue evaluation device 100 may be used. Hereinafter, each unit will be described.

(Control unit 30)

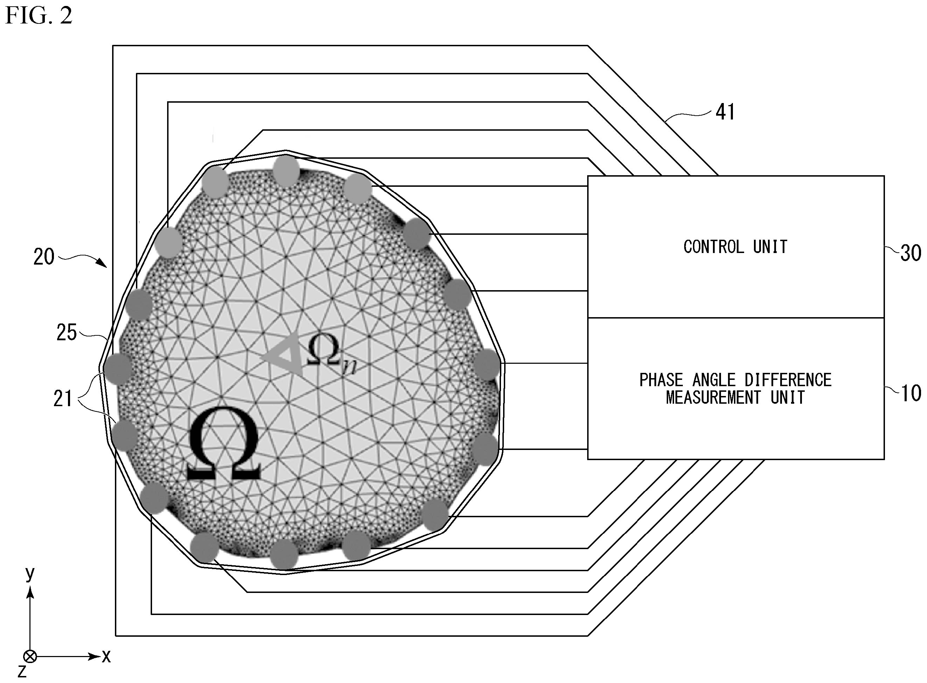

The control unit 30 will be described with reference to FIG. 2. FIG. 2 is a schematic diagram showing the phase angle variation amount measurement unit 10 shown in FIG. 1, a method for measuring a phase angle difference by the phase angle variation amount measurement unit 10, and the control unit 30. FIG. 2 is a schematic diagram of the control unit 30 in a state in which the sensor 20 is placed on the leg of the subject. Here, a case where the sensor is in contact with the leg of the subject will be described as an example, but the measurement site of the subject is not limited to the leg in the present invention. The control unit 30 is configured of, for example, a current injection device that injects a current (or a voltage injection device that injects a voltage) (not shown), and a multiplexer (not shown) for switching wirings connected to each electrode. An impedance analyzer or the like may be used as the current injection device or the voltage injection device. Here, the impedance analyzer has a function of injecting a current or a voltage by changing an applied frequency and an amplitude.

As shown in FIG. 2, the control unit 30 is connected to the electrodes 21 by one or more sensors 20 and electric wires 41. The sensor 20 includes a plurality of electrodes 21 (shown as an example with the number of electrodes Q = 16) placeable on skin of the subject spaced apart from each other, and a support 25 that holds the electrodes 21. The control unit 30 uses one or more sensors 20 to inject a predetermined current or voltage between the electrodes 21 in a state in which each electrode 21 of the sensor 20 is in contact with the skin of the subject.

The multiplexer has a function of determining a current-injection voltage-measurement pattern (or a voltage-injection current-measurement pattern). That is, two electrodes are selected from the electrodes, and a current or a voltage is injected. Furthermore, as described in the phase angle variation amount measurement unit 10 described later, two electrodes are selected from the electrodes, and a voltage or a current is measured.

In a case of injecting a current, a current is injected and a voltage is measured based on a predetermined current-injection voltage-measurement pattern (a pattern in which two electrodes are sequentially selected from a large number of electrodes and a current is injected to sequentially measure a voltage). In a case of injecting a voltage, a voltage is injected and a current is measured based on a predetermined voltage-injection current-measurement pattern (a pattern in which two electrodes are sequentially selected from a large number of electrodes and a voltage is injected to sequentially measure a current).

Hereinafter, in a case of injecting a current and measuring a voltage, the features of the present invention will be described mainly focusing on the current-injection voltage-measurement pattern as a center, and the detailed description in a case of injecting a voltage and measuring a current may be omitted.

Examples of the current-injection voltage-measurement pattern include an adjacent method, a counter electrode method, a reference method, and the like.

The control unit 30 may use, for example, a CPU to execute a predetermined program and control the multiplexer, the current injection device (or the voltage injection device, the impedance analyzer).

Here, the number Q of the electrodes 21 is, for example, 3 or more, and the number Q of the electrodes 21 is preferably 8 or more. In particular, the upper limit number is not limited, but the number Q of the electrodes 21 is preferably 32 or less. The number Q of the electrodes 21 may be 16 or less. To improve the accuracy of the concentration distribution, it is preferable that the number of electrodes is large. The placement position of the electrodes 21 is not particularly limited. The electrodes 21 may be placed to surround the periphery of the subject (here, the periphery of the leg), some electrodes may be placed to be biased without surrounding the periphery, and the inter-electrode distance may be equal or unequal. The bias or the inequality of these electrodes is corrected in the calculation of the Jacobian matrix described later.

The electrodes 21 are electrically connected to the control unit 30. The material and the shape of the electrodes 21 are not particularly limited as long as the current or the voltage can be injected to the skin of the subject. Examples of the material of the electrodes 21 include metals such as Au, Ag, Cu, and stainless steel, conductive polymers, fibers having surfaces coated with a metal, and fibers having surfaces coated with a conductive polymer. The shape of the electrodes 21 on the surface perpendicular to the current injection direction is not particularly limited to bring each electrode 21 into contact with the skin, but is, for example, a circular shape or a polyangular shape. The electrodes 21 are preferably non-invasive electrodes.

The electrical connection method between the electrodes 21 and the control unit 30 is not particularly limited, and a known electrical connection method can be used. In the present embodiment, each electrode 21 and the control unit 30 are connected by an electric wire 41.

The support 25 is not particularly limited as long as the electrodes 21 can be held. It is preferable that the support 25 can place the electrodes 21 on the skin of the subject. Here, the expression "placeable on the skin of the subject" means that each electrode 21 is placed to be in contact with the skin when the subject wears the sensor 20.

It is preferable that the support 25 can inject a predetermined pressure to the extent that the electrodes 21 can be closely attached to the subject. As a result, the adhesiveness between the electrodes 21 and the subject is improved, and the current or the voltage can be more accurately injected and the voltage or the current can be measured. The material of the support 25 is not particularly limited, and for example, an insulator such as an elastomer, leather, or cloth is preferable. The shape of the support 25 is not particularly limited, and examples thereof include a band shape and the like.

(Phase angle variation amount measurement unit 10)

The phase angle variation amount measurement unit 10 will be described with reference to FIG. 2. The phase angle variation amount measurement unit 10 measures a feature amount obtained based on a variation amount of a phase angle for a current-injection voltage-measurement pattern or a voltage-injection current-measurement pattern. The local biological tissue evaluation device 100 according to the present embodiment has a significant feature in that, as described in the control unit 30 described above, the phase angle variation amount measurement unit 10 selects two electrodes among the electrodes by the current-injection voltage-measurement pattern (or the voltage-injection current-measurement pattern) determined by the multiplexer, measures the voltage or the current, and measures, for example, the phase angle difference. The phase angle difference is an example of the feature amount. The phase angle variation amount measurement unit 10 includes, for example, a voltage-measurement device (not shown) and a current-measurement device (not shown) that can accurately measure a time change in the voltage and the current, and measures the phase angle difference. An impedance analyzer may be used as the voltage-measurement device and the current-measurement device. The impedance analyzer is a device that changes an applied frequency and an amplitude, measures a measured voltage (injected voltage) and an injected current (measured current), and measures a phase angle difference therebetween. In particular, a ratio (V/I) of the measured voltage V to the injected current I is referred to as an impedance Z, a real part thereof is referred to as a resistance Z', and an imaginary part thereof is referred to as a reactance Z", and the following description will be made using these terms.

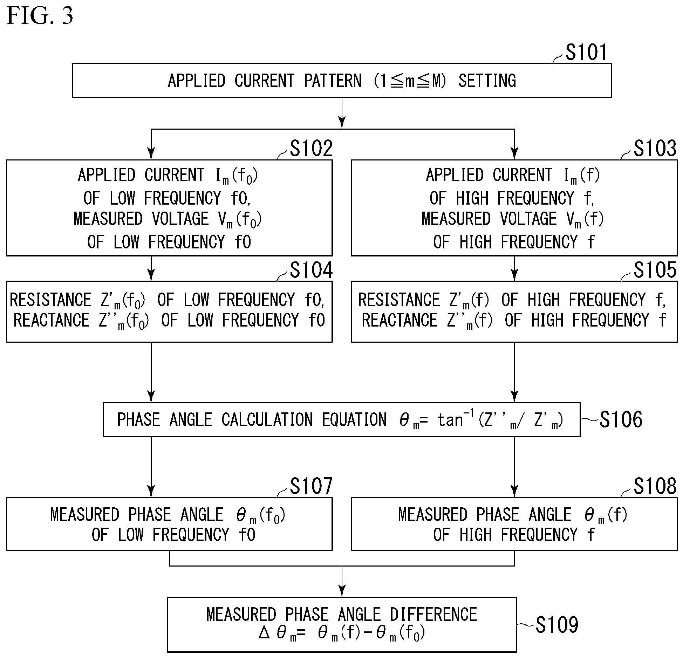

Here, a method for measuring the measured phase angle difference $\Delta\theta_m$ will be described with reference to FIG. 3. FIG. 3 is a flowchart showing a flow of measuring the measured phase angle difference $\Delta\theta_m$ based on the current-injection voltage-measurement pattern. First, an injected current pattern ($1 \leq m \leq M$) is set (S101), using at least two applied frequencies (a low frequency is denoted by $f_0$, and a high frequency is denoted by f), an injected current $I_m(f_0)$ of the low frequency $f_0$ is injected and a measured voltage $V_m(f_0)$ of the low frequency $f_0$ is measured (S102), and further, an injected current $I_m(f)$ of the high frequency f is injected and a measured voltage $V_m(f)$ of the high frequency f is measured (S103). At this time, a temporal deviation occurs between the injected current and the measured voltage. Therefore, a resistance $Z'_m(f_0)$ at the low frequency $f_0$, a reactance $Z''_m(f_0)$ at the low frequency $f_0$, a resistance $Z'_m(f)$ at the high frequency f, and a reactance $Z''_m(f)$ at the high frequency f are obtained (S104, S105).

Next, a measured phase angle $\theta_m(f_0)$ at the low frequency $f_0$ and a measured phase angle $\theta_m(f)$ at the high frequency f are determined by using a phase angle calculation equation $\theta_m = \tan^{-1}(Z''_m/Z'_m)$ (S106, S107, S108). A measured phase angle difference $\Delta\theta_m$ is measured from the determined measured phase angle $\theta_m(f_0)$ at the low frequency $f_0$ and the determined measured phase angle $\theta_m(f)$ at the high frequency f, $\Delta\theta_m = \theta_m(f) - \theta_m(f_0)$ (S109).

The present embodiment is particularly characterized in that the measured phase angle difference $\Delta\theta_m$ is measured in this manner.

Hereinafter, in the local biological tissue evaluation device 100 of the present embodiment, an example in which a voltage is measured using the adjacent method will be described. It is noted that a measurement example for one sensor 20 will be described below, but the measurement can be performed in the same manner even in a case where there are two or more sensors 20.

It is noted that the phase angle variation amount measurement unit 10 may control the control unit 30 only inside the phase angle variation amount measurement unit 10 and perform the impedance measurement, or may control the control unit 30 according to a program executed by the calculation evaluation unit 50 and perform the impedance measurement. The result of the impedance measurement is sent to the calculation evaluation unit 50. A method for transmitting information to the calculation evaluation unit 50 is not particularly limited. The information may be sent from the control unit 30 to the calculation evaluation unit 50 in a wired manner, or may be sent to the calculation evaluation unit 50 by other transmission means.

(Phase angle Jacobian matrix creation unit 51)

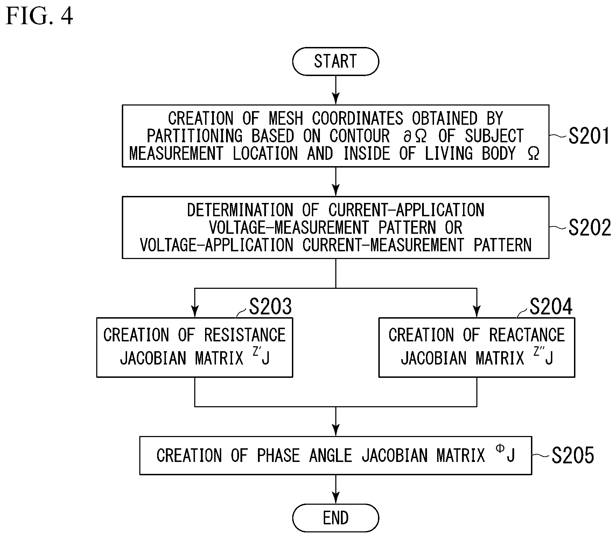

The phase angle Jacobian matrix creation unit 51 creates a phase angle Jacobian matrix based on the current-injection voltage-measurement pattern or the voltage-injection current-measurement pattern. FIG. 4 is a flowchart for creating a phase angle Jacobian matrix $^{\Phi}J$ in the phase angle Jacobian matrix creation unit. The phase angle Jacobian matrix (phase angle sensitivity matrix) is a sensitivity matrix that indicates, as an angle, how much the measured voltage when a current is injected (or the measured current when a voltage is injected) is delayed or advanced in time with respect to a change in the electrical properties (conductivity or permittivity) distributed in space relative to a reference, and needs to be determined in advance as a known value. The phase angle Jacobian matrix of the subject varies depending on the spatial distribution of the electrical properties of the subject, the body shape, and the like, and the phase angle distribution can be calculated once the phase angle Jacobian matrix of the subject is known.

As shown in FIG. 4, as a procedure for creating the phase angle Jacobian matrix $^{\Phi}J$, first, mesh coordinates obtained by partitioning based on the contour $\partial\Omega$ of the subject measurement location and the inside of the living body $\Omega$, which are measured or estimated in advance, are created (S201). Here, the inside of the living body of the subject indicates the size of a muscle compartment, a bone, a fat, and the like in the inside of the limbs, the inside of the trunk, and the like. The contour $\partial\Omega$ and the inside of the living body $\Omega$ may be such that (1) mesh coordinates may be created based on X-ray images, MRI images, or the like of the inside of the living body of the subject, such as inside of limbs and inside of a trunk, captured in advance, (2) mesh coordinates may be created based on X-ray images or an MRI image database of the inside of the living body, such as the average limbs or the trunk, which takes into account the gender, the nationality, the age, the weight, the height, the BMI, and the like, and furthermore, (3) X-ray images or the MRI image database of (2) may be customized based on the features of the subject itself, such as the BMI, and the mesh coordinates may be created by assuming that the customized database is X-ray images or MRI images of the inside of the living body of the subject itself.

Next, the current-injection voltage-measurement pattern (or the voltage-injection current-measurement pattern) is determined based on the contour $\partial\Omega$ (S202). The selection of two electrodes for injecting a current and two electrodes for measuring a voltage is considered in m ($1 \leq m \leq M$) patterns. Here, M is the total number of measurement patterns. Here, a relationship between m and i, j, k, and l used as superscripts below will be described. The characters i, j, k, and l mean that a current is injected between the i-th and j-th electrodes and a voltage is measured between the k-th and l-th electrodes, and this pattern is referred to as an m-th measurement pattern.

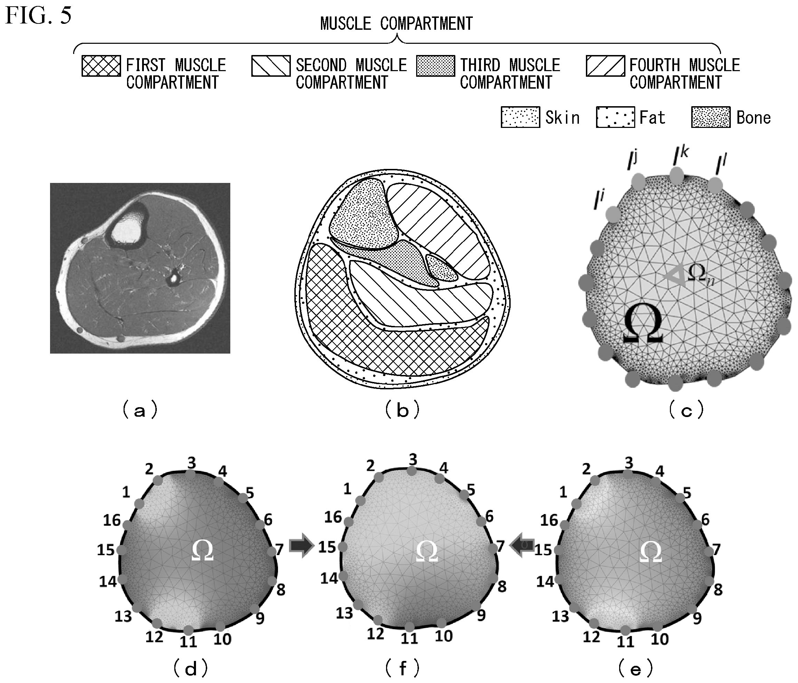

Next, the resistance Jacobian matrix $^{Z'}J$ and the reactance Jacobian matrix $^{Z''}J$ are created (S203, S204). Here, there are various methods for obtaining the reactance Jacobian matrix $^{Z''}J^m_n$ and the resistance Jacobian matrix $^{Z'}J^m_n$ in the n-th mesh in the m-th injection measurement pattern, and Geselowitz's equation represented by Equation (1) can be used. Here, Z on the left side indicates an impedance that is a complex number, and includes a resistance (resistive) component (real part of the complex impedance) Z' and a reactance component (imaginary part of the complex impedance) Z''. n indicates a mesh number, and i, j, k, and l correspond to the m-th measurement pattern. With reference to FIG. 5 described later, i and j are electrodes to which a current is injected, and k and l are electrodes at which a voltage is measured.

Furthermore, for the right side of Equation (1) below, u indicates an electric potential (potential) of each mesh number n, $\nabla$ is called a differential operator (nabla) and indicates a differential in a space, $u(I^{i,j})$ is a distribution of an electric potential (potential) in a field when a current is injected between the i-th and j-th electrodes, and $u(I^{k,l})$ is a distribution of an electric potential (potential) in a field when a current is injected between the k-th and l-th electrodes. $I^{i,j}$ is an injected current between the i-th electrode $e^i$ and the j-th electrode $e^j$ (i = 1, 2, ..., E-1, j = i+1, i+2, ..., E (i $\neq$ j)), and $I^{k,l}$ means an injected current between the k-th electrode $e^k$ and the l-th electrode $e^l$. $\nabla u(I^{i,j})$ and $\nabla u(I^{k,l})$ mean a gradient of each electric potential (that is, an electric field), which is a vector value, and since $\cdot$ indicates an inner product, this operation results in a scalar value. By integrating this scalar value in the cross-sectional field of the living body $\Omega$, the resistance Jacobian matrix $^{Z'}J^m_n$ and the reactance Jacobian matrix $^{Z''}J^m_n$ can be obtained. $\Omega$ in Equation (2) above may be a two-dimensional region or a three-dimensional region, and in a case of a two-dimensional region, the integration indicates a surface integral, and in a case of a three-dimensional region, the integration indicates a volume integration.

Finally, the phase angle Jacobian matrix $^{\Phi}J^m_n$ is created (S205). Here, there are several methods for obtaining the element $^{\Phi}J^m_n$ of the phase angle Jacobian matrix in the n-th mesh in the m-th measurement pattern, and as an example thereof, a method of obtaining the element $^{\Phi}J^m_n$ by dividing the reactance Jacobian matrix $^{\Phi}J^m_n$ by the resistance Jacobian matrix $^{Z'}J^m_n$ will be described. The element $^{\Phi}J^m_n$ of the phase angle Jacobian matrix in the n-th mesh in the m-th measurement pattern is represented by Equation (2).

$$^{Z}J_n^{i,j,k,l} = -\int_{\Omega} \nabla u\left(I^{i,j}\right) \bullet \nabla u\left(I^{k,l}\right) d\Omega \tag{1}$$

$$^{\Phi}J_n^m = \frac{^{Z''}J_n^m}{^{Z'}J_n^m} \tag{2}$$

As described above, the phase angle Jacobian matrix creation unit 51 creates the mesh coordinates of the subject measurement location and a relational equation of the n-th mesh in the m-th measurement pattern, and derives the current and the electric potential at each mesh coordinate, and the phase angle of the impedance, which is a ratio of the voltage to the current, as the element of the phase angle Jacobian matrix as a solution of the equation. The above description is for the element of the phase angle Jacobian matrix in the n-th mesh in the m-th measurement pattern, but by repeating this process, the phase angle Jacobian matrix $^{\Phi}J$ of the inside of the living body $\Omega$ of the subject can be created. When the element $^{\Phi}J^m_n$ of the phase angle Jacobian matrix of Equation (2) is obtained for all meshes n ($1 \leq n \leq N$) and all measurement patterns m ($1 \leq m \leq M$), the phase angle Jacobian matrix $^{\Phi}J$ can be represented by a matrix as in Equation (3), and $^{\Phi}J_n$ of the n-th mesh in Equation (3) below is represented by Equation (4). Equation (4) is a column vector $^{\Phi}Jn$, and T in Equation (4) means a transpose.

$$^{\Phi}\mathbf{J}=\left[^{\Phi}\mathbf{J}_1,\ldots,\ ^{\Phi}\mathbf{J}_n\ldots,\ ^{\Phi}\mathbf{J}_N\right]\in\mathbb{R}^{M\times N} \tag{3}$$

$$^{\Phi}\mathbf{J}_n=\left[^{\Phi}J_1,\ldots,\ ^{\Phi}J_m,\ldots,\ ^{\Phi}J_M\right]^{T}\in\mathbb{R}^{M} \tag{4}$$

Regarding how to operate the phase angle Jacobian matrix $^{\Phi}J$, a large number of shapes or positions of muscle compartments may be predicted and calculated in advance and stored as a database, an appropriate phase angle Jacobian matrix $^{\Phi}J$ may be selected from the database, a new phase angle Jacobian matrix $^{\Phi}J$ may be created by combining several phase angle Jacobian matrices $^{\Phi}J$ from the database using machine learning or the like, and the phase angle Jacobian matrix $^{\Phi}J$ of the subject may be created on the spot without using the database or the like.

The created phase angle Jacobian matrix $^{\Phi}J$ is sent to the local biological tissue evaluation unit 60 via the image reconstruction unit 52. In the following description, the calf will be described as an example, but the present invention is not limited to the analysis of the interior of the calf.

As in the method for creating the mesh coordinates described in (1) above, a method for creating the phase angle Jacobian matrix $^{\Phi}J$ will be described in more detail by taking, as an example, a method for creating the phase angle Jacobian matrix $^{\Phi}J$ based on an MRI image of the calf of the subject itself captured in advance. (a) of FIG. 5 is an image showing a cross-sectional structure of the calf in an MRI cross-sectional image of the calf of the subject. From this figure, the contour $\partial\Omega$ and the mesh number and position of the inside of the living body $\Omega$ are determined in accordance with (S201) in FIG. 4. (b) of FIG. 5 is a diagram in which the cross-sectional position of the element in the calf is classified from the image in (a) of FIG. 5. As shown in (b) of FIG. 5, the calf is composed of skin, fat, bone, and four muscle compartments divided into large sections. Specifically, to evaluate the quality of the local biological tissue in the calf, for example, as shown in (b) of FIG. 5, it is preferable to classify the four muscle compartments into a first muscle compartment (gastrocnemius muscle), a second muscle compartment (soleus muscle), a third muscle compartment (tibialis posterior muscle, flexor digitorum longus muscle, and flexor hallucis longus muscle), and a fourth muscle compartment (anterior portion, extensor digitorum longus muscle, and fibularis longus muscle), and the classification may be further detailed than the four muscle compartments.

(c) of FIG. 5 is a diagram showing an example of the determined mesh number and position of the inside of the living body $\Omega$ obtained by determining the placement position of the electrode 21 in accordance with (S202) in FIG. 4 with respect to the contour $\partial\Omega$ determined in (S201). For example, based on the MRI image shown in (a) of FIG. 5, the electrode positions (denoted by ● marks) on the surface of the subject are determined as shown in (c) of FIG. 5, and a mesh is further created. The electrode positions may be placed at equal intervals or non-equal intervals on the surface of the living body.

(d) of FIG. 5 is an example of the resistance Jacobian matrix $^{Z'}J$ of the calf in the m-th measurement pattern. Specifically, with respect to the mesh determined in (S202), the frequency-dependent conductivity and permittivity are assigned to each of the first muscle compartment to the fourth muscle compartment, and, in accordance with (203), using Equation (1), the resistance Jacobian matrix $^{Z'}J$ of the resistance (resistive) component (real part of the complex impedance) Z' of the impedance is obtained.

(e) of FIG. 5 is an example of the reactance Jacobian matrix $^{Z''}J$ of the calf in the m-th measurement pattern. Specifically, with respect to the mesh determined in (S202), the frequency-dependent conductivity and permittivity are assigned to each of the first muscle compartment to the fourth muscle compartment, and, in accordance with (204), using Equation (1), the reactance Jacobian matrix $^{Z''}J$ of the reactance component (imaginary part of the complex impedance) Z" of the impedance is obtained.

(f) of FIG. 5 is an example of the phase angle Jacobian matrix $^{\Phi}J$ of the calf in the m-th measurement pattern. The phase angle Jacobian matrix $^{\Phi}J$ is obtained in accordance with (S205) using Equation (2).

(f) of FIG. 5 shows the phase angle Jacobian matrix of the calf in the m-th measurement pattern, and all M measurement patterns are stored in the phase angle Jacobian matrix $^\Phi J$ of Equation (3).

(Image reconstruction unit 52)

The image reconstruction unit 52 images the phase angle distribution of the inside of the living body of the subject using the feature amount measured by the phase angle variation amount measurement unit 10, and the phase angle Jacobian matrix. For example, the phase angle Jacobian matrix creation unit and the image reconstruction unit 52 image the phase angle distribution $\Phi$ using the measured phase angle difference $\Delta\theta_m$ and the phase angle Jacobian matrix $^\Phi J_n$. The phase angle distribution $\Phi$ may be represented as a matrix having the phase angle $\Phi_n$ of the n-th mesh as an element as shown in Equation (5). Here, a method for imaging the phase angle distribution $\Phi$ using the phase angle Jacobian matrix $^\Phi J$ based on the Gauss-Newton method will be described, but the present invention is not limited to the Gauss-Newton method.

A relationship between the phase angle distribution $\Phi^i$ of the i-th repetition and the phase angle distribution $\Phi^{i+1}$ of the (i+1)-th repetition by the Gauss-Newton method is represented by Equation (6). In Equation (6), R is a regularization matrix, $\lambda$ is a relaxation coefficient scalar (for example, obtained by the L-curve method), $\Delta\theta$ is the measured phase angle difference represented by Equation (7), and is the measured phase angle difference between the selected low frequency $f_0$ and the selected high frequency f. In Equation (7), m ($1 \leq m \leq M$) means the m-th one in the total number M of the current-injection voltage-measurement patterns (or the voltage-injection current-measurement patterns). The measured phase angle difference $\Delta\theta_m$ of the m-th pattern is represented by Equation (8). In the present invention, the phase angle distribution $\Phi$ of the inside of the living body of the subject is imaged as a preliminary stage for evaluating the quality of the local biological tissue. For example, in a case where a muscle is taken as an example of the local biological tissue, since the value of the phase angle distribution $\Phi$ is large in a case where the amount of fat in the muscle is small and the muscle density is high, it can be evaluated that the quality of the muscle is higher in the muscle compartment having a large phase angle distribution $\Phi$ as a whole. This is merely a case where the muscle is taken as an example of the local biological tissue, and the evaluation of the quality of the local biological tissue based on the phase angle difference can be appropriately changed according to the target local biological tissue.

$$\Phi = \begin{bmatrix} \Phi_1, \ldots, \Phi_n, \ldots, \Phi_N \end{bmatrix}^T \in \mathbb{R}^N \tag{5}$$

$$\Phi^{i+1} = \Phi^i - \left( {}^\Phi\mathbf{J}^T\ {}^\Phi\mathbf{J} + \lambda\mathbf{R} \right)^{-1}\ {}^\Phi\mathbf{J}^T\ \Delta\theta \tag{6}$$

$$\Delta\theta = \begin{bmatrix} \Delta\theta_1, \ldots, \Delta\theta_m, \ldots, \Delta\theta_M \end{bmatrix}^T \in \mathbb{R}^M \tag{7}$$

$$\Delta\theta_m(f - f_0) = \theta_m(f) - \theta_m(f_0) \tag{8}$$

The phase angle distribution $\Phi$ of the subject imaged by the image reconstruction unit 52 is transmitted to the local biological tissue evaluation unit 60.

(Local biological tissue evaluation unit 60)

The local biological tissue evaluation unit 60 includes at least any one of the local biological tissue aging evaluation unit 53, the local biological tissue fatty infiltration evaluation unit 54, or the local biological tissue cross-sectional area evaluation unit 55. Hereinafter, each unit of the local biological tissue evaluation unit 60 will be described. In the following, there is a place where the muscle is described as an example of the biological tissue, but the present invention is not limited to only the muscle. In addition, in a case where the muscle is taken as an example, the term, muscle, may be used in expression such as muscle aging and muscle cross-sectional area, but in the present invention, the terms can be replaced with general aging and cross-sectional area.

(Local biological tissue aging evaluation unit 53)

To quantitatively evaluate the muscle aging (MA), the local biological tissue aging evaluation unit 53 calculates the spatial mean of the phase angle distribution $\Phi$ from the phase angle distribution $\Phi$ obtained by imaging. For example, the spatial mean of the phase angle distribution $\Phi$ (spatial mean phase angle $<\Phi>$) may be calculated from Equation (9). Hereinafter, $<>$ may be used as a symbol of the spatial mean. In Equation (9), n indicates a mesh number, and N indicates the sum of the meshes. The muscle aging, which is one index of the quality of the muscle, can be evaluated at a local position where the sensor is placed, not the entire region in the related art, by the magnitude of the spatial mean phase angle $<\Phi>$.

(Local biological tissue fatty infiltration evaluation unit 54)

To quantitatively evaluate the fatty infiltration (FI), the local biological tissue fatty infiltration evaluation unit 54 assigns the phase angle distribution $\Phi$ of the subject imaged by the image reconstruction unit 52 to each local biological tissue of the subject. For example, in a case where the local fatty infiltration (FI) of the muscle compartment is evaluated, the phase angle distribution $\Phi$ is assigned to each muscle compartment. The method for assigning to each local biological tissue will be described using Equation (10). In Equation (10), the subscript p ($1 \leq p \leq P$) is the number of the muscle compartment, and as described above, in a case where the muscle compartment of the calf is taken as an example, P (the maximum value of p) is desirably about 4, and specifically, p = 1 can be the first muscle compartment (gastrocnemius muscle), p = 2 can be the second muscle compartment (soleus muscle), p = 3 can be the third muscle compartment (tibialis posterior muscle, flexor digitorum longus muscle, and flexor

hallucis longus muscle), and p = 4 can be the fourth muscle compartment (anterior portion, extensor digitorum longus muscle, and fibularis longus muscle). $n_p$ is the mesh number in the p-th muscle compartment, and $N_p$ indicates the total number of meshes in the p-th muscle compartment. It is preferable that the local biological tissue fatty infiltration evaluation unit 54 assigns the phase angle distribution $\Phi$ to each local biological tissue (for example, the muscle compartment of the calf) as in Equation (10), and calculates the spatial mean of the phase angle distribution $\Phi$ in each local biological tissue (for example, in the muscle compartment). In a case where the local biological tissue fatty infiltration evaluation unit 54 evaluates the fatty infiltration in the muscle compartment, for example, as shown in Equation (11), the quality of each local biological tissue can be evaluated by calculating the spatial mean phase angles $<\Phi_{p=1}>$, $<\Phi_{p=2}>$, $<\Phi_{p=3}>$, and $<\Phi_{p=4}>$ of the four muscle compartments. The local biological tissue fatty infiltration evaluation unit 54 transmits the phase angle distribution $\Phi_p$ of the local biological tissue and the spatial mean phase angle $<\Phi_p>$ of each local biological tissue to at least one of the local biological tissue cross-sectional area evaluation unit 55 or the output unit 56.

$$\langle \Phi \rangle = \frac{\sum_{n=1}^{n=N} \Phi_n}{N} \tag{9}$$

$$\Phi_p = \left[ \Phi_1, \dots, \Phi_{n_p}, \dots, \Phi_{N_p} \right]^T \in \mathbb{R}^{N_p} \tag{10}$$

$$\langle \Phi_p \rangle = \frac{\sum_{n_p=1}^{n_p=N_p} \Phi_{n_p}}{N_p} \tag{11}$$

(Local biological tissue cross-sectional area evaluation unit 55)

To quantitatively evaluate the biological tissue cross-sectional area ratio, the local biological tissue cross-sectional area evaluation unit 55 performs binarization processing on the local biological tissue phase angle distribution $\Phi_p$ and calculates the local biological tissue cross-sectional area ratio. The binarization processing of the local biological tissue cross-sectional area ratio is not particularly limited for the target local biological tissue. Examples of the binarization processing include methods such as otsu binarization method, Kittler-Illingworth method, K-means method, and fuzzy clustering method. Here, a method for calculating the local biological tissue cross-sectional area ratio by binarization processing based on the otsu binarization method will be described as an example. The otsu binarization method is a problem of searching for a threshold value that minimizes the intra-class variance defined as a weighted sum of the variances of two classes.

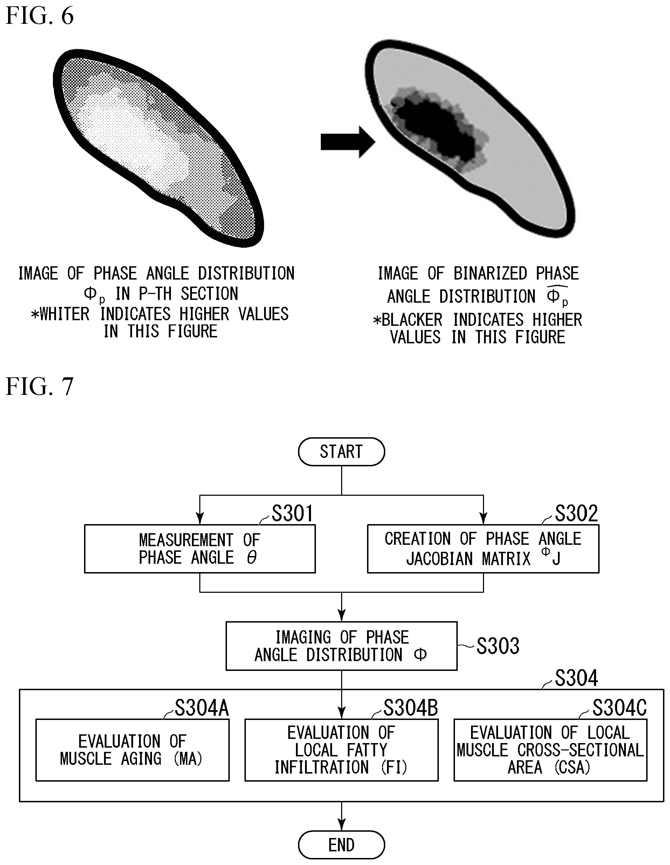

FIG. 6 shows an example of an image of the phase angle distribution $\Phi_p$ in the p-th compartment and an image of the binarized phase angle distribution $\Phi_p{}^\wedge$, and the symbol $^\wedge$ indicates binarization. A process of calculating the binarized phase angle distribution $\Phi_p{}^\wedge$ from the phase angle distribution $\Phi_p$ in the p-th compartment will be described. First, for example, an optimal threshold value $\varepsilon^*$ is determined, and the value of $\Phi_p$ in the $n_p$-th mesh in the p-th compartment is converted into a binary value (a value of a high-intensity phase angle and a value of a low-intensity phase angle) based on the optimal threshold value $\varepsilon^*$ as in Equation (12). In Equation (12), $n_p$ is the n-th mesh number in the p-th compartment, and is an integer of $1 \leq n_p \leq N_p$.

Before determining the optimal threshold value $\varepsilon^*$, the count function $\alpha$ will be described. For example, the count function $\alpha$ has two types of arguments. The first type is a function for counting the total number of meshes of $\Phi_p$ equal to or less than $\varepsilon$, is represented by $\alpha(\Phi_p{}^\wedge\{\Phi n_p < \varepsilon\})$, and is referred to as a first count function. The second type is a function for counting the total number of meshes of $\Phi_p$ greater than $\varepsilon$, and is represented by $\alpha(\Phi_p{}^\wedge\{\Phi n_g > \varepsilon\})$, and is referred to as a second count function. That is, the first count function $\alpha$ indicates the total number of meshes of $\Phi_p$ in a case of $\Phi n_p < \varepsilon$, and the second count function $\alpha$ indicates the total number of meshes of $\Phi_p$ in a case of $\Phi n_p > \varepsilon$.

Next, the intensity dispersion $\gamma(\varepsilon)$ as a function of the threshold value variable $\varepsilon$ will be described. The threshold value variable $\varepsilon$ in the parentheses is a function of $\gamma$. The intensity dispersion $\gamma$ is represented by Equation (17). $\alpha_0(\varepsilon)$ in Equation (17) is represented by the Equation (13), and is a weight indicating a mesh occupancy rate indicating a value equal to or less than $\varepsilon$, which is obtained by dividing the first count function $\alpha$ by the total number of meshes in the p-th compartment. $\alpha_1(\varepsilon)$ in Equation (17) is represented by the Equation (15), and is a weight indicating a mesh occupancy rate indicating a value equal to or more than $\varepsilon$, which is obtained by dividing the second count function $\alpha$ by the total number of meshes in the p-th compartment. $\beta_0(\varepsilon)$ in Equation (17) is represented by the following Equation (14), and indicates a class variance indicating a value equal to or less than $\varepsilon$. $\beta_1(\varepsilon)$ in Equation (17) is represented by the following Equation (16), and indicates a class variance indicating a value equal to or more than $\varepsilon$. In the end, the intensity dispersion $\gamma(\varepsilon)$ as a function of the threshold value variable $\varepsilon$ is represented by Equation (17).

$$\widehat{\Phi}_p = \begin{cases} = 0, \text{ in the case of } \Phi_{n_p} < \varepsilon^* \left(\text{low intensity}\right) \\ = 1, \text{ in the case of } \Phi_{n_p} \geq \varepsilon^* \left(\text{high intensity}\right) \end{cases} \tag{12}$$

$$\alpha_0\left(\varepsilon\right) = \left(\frac{\alpha\left(\widehat{\Phi}_p \left\{\Phi_{n_p} < \varepsilon\right\}\right)}{N_p}\right) \tag{13}$$

$$\beta_0(\varepsilon) = \left(\frac{\sum_{n_p}^{N_p} \left(\Phi_{n_p} - \alpha_0(\varepsilon)\right)^2}{N_p - 1}\right) \tag{14}$$

$$\alpha_1\left(\varepsilon\right) = \left(\frac{\alpha\left(\widehat{\Phi}_p \left\{\Phi_{n_p} > \varepsilon\right\}\right)}{N_p}\right) \tag{15}$$

$$\beta_1(\varepsilon) = \left( \frac{\sum_{n_p}^{N_p} \left( \Phi_{n_p} - \alpha_1(\varepsilon) \right)^2}{N_p - 1} \right) \tag{16}$$

$$\gamma(\varepsilon) = \alpha_0(\varepsilon)\beta_0(\varepsilon) + \alpha_1(\varepsilon)\beta_1(\varepsilon) \tag{17}$$

$$\varepsilon^* = \operatorname{argmax} \sum_{x=0}^{X} \sum_{\varepsilon=x\Delta\varepsilon}^{\varepsilon=1} \gamma(\varepsilon) \tag{18}$$

The optimal threshold value $\varepsilon^*$ is represented by, for example, Equation (18) using the intensity dispersion $\gamma(\varepsilon)$. In this case, the threshold value variable $\varepsilon$ at which the double sum of the intensity dispersion $\gamma(\varepsilon)$ is maximized is defined as the optimal threshold value $\varepsilon^*$. Here, the threshold value variable $\varepsilon$ is represented by $\varepsilon = x\Delta\varepsilon$ using a minute threshold value $\Delta\varepsilon$ appropriately determined and the number of steps x $(1 \leq x \leq X)$, and $\varepsilon$ can take a value of $(0 \leq \varepsilon \leq 1)$.

Finally, the local biological tissue cross-sectional area ratio (here, the local muscle cross-sectional area ratio) $\varphi$ is obtained from the binarized phase angle distribution $\Phi_p^{\wedge}$ using Equation (19). In Equation (19), A is a function for converting the number of meshes in ( ) into an area, $\Phi n_p \geq \varepsilon^*$ in the numerator in ( ) indicates the number of meshes indicating the high-intensity phase angle distribution in the p-th compartment, and $\Phi_{Np}$ in the denominator in ( ) indicates the total number of meshes in the p-th compartment. In the end, the left side $\varphi$ of the equation indicates the cross-sectional area ratio of the local biological tissue. The cross-sectional area ratio $\varphi$ of the local biological tissue is sent to the output unit 56.

$$\varphi = \frac{A\left( \varphi_p \left\{ \Phi_{n_p} > \varepsilon^* \right\} \right)}{A\left( \varphi_p \right)} \tag{19}$$

(Output unit 56)

The output unit 56 outputs at least one of the spatial mean phase angle $\langle\Phi\rangle$ calculated by the local biological tissue aging evaluation unit 53, the phase angle distribution $\Phi_p$ of the local biological tissue and the spatial mean phase angle $\langle\Phi_p\rangle$ of each local biological tissue calculated by the local biological tissue fatty infiltration evaluation unit 54, or the cross-sectional area ratio of the local biological tissue calculated by the local biological tissue cross-sectional area evaluation unit 55. The output destination may be a display unit such as a liquid crystal display, or a storage device such as an HDD.

(Local biological tissue evaluation method)

Next, a local biological tissue evaluation method using the local biological tissue evaluation device 100 will be described. FIG. 7 shows a flowchart of the local biological tissue evaluation method according to the present embodiment. The local biological tissue evaluation method according to the present embodiment includes a first step S301 of using a sensor including a plurality of electrodes 21 placeable on skin of a subject spaced apart from each other, injecting a current or a voltage between the electrodes 21 in a state in which each of the electrode 21 is in contact with the skin of the subject, measuring a voltage based on a current-injection voltage-measurement pattern when the current is injected, measuring a current based on a voltage-injection current-measurement pattern when the voltage is injected between the electrodes 21, and measuring a feature amount obtained based on a variation amount of a phase angle for the current-injection voltage-measurement pattern or the voltage-injection current-measurement pattern, for example, a measured phase angle difference $\Delta\theta$, a second step S302 of creating a phase angle Jacobian matrix $\Phi J$ based on the current-injection voltage-measurement pattern or the voltage-injection current-measurement pattern, and a third step S303 of imaging a phase angle distribution $\Phi$ in the biological tissue of the subject from the measured phase angle difference $\Delta\theta$ and the phase angle Jacobian matrix $\Phi J$. The local biological tissue evaluation method according to the present embodiment may further include a fourth step S304 of evaluating the local biological tissue using the imaged phase angle distribution $\Phi$.

(First step S301)

In the first step S301, the impedance of the subject is measured using the phase angle variation amount measurement unit 10. Specifically, a sensor including a plurality of electrodes 21 placeable on the skin of the subject spaced apart from each other is used, a current or a voltage is injected between the electrodes 21 in a state in which each of the electrodes 21 is in contact with the skin of the subject, the voltage is measured based on a current-injection voltage-measurement pattern when the current is injected, and a current is measured based on a voltage-injection current-measurement pattern when the voltage is injected. Then, the measured phase angle difference $\Delta\theta$ is measured.

(Second step S302)

In the second step S302, the phase angle Jacobian matrix $^\Phi J$ is created using the phase angle Jacobian matrix creation unit 51 based on the current-injection voltage-measurement pattern or the voltage-injection current-measurement pattern.

(Third step S303)

In the third step S303, the phase angle distribution $\Phi$ of the inside of the living body of the subject is imaged using the image reconstruction unit 52 from the measured phase angle difference $\Delta\theta$ measured in the first step and the phase angle Jacobian matrix $^\Phi J$ obtained in the second step.

(Fourth Step S304)

In the fourth step, the biological tissue is evaluated using the phase angle distribution imaged in the third step. In the fourth step, for example, at least one of the following three types of processing is performed.

(First processing S304A of fourth step)

In the first processing S304A of the fourth step, the muscle aging is evaluated using the local biological tissue aging evaluation unit 53 from the phase angle distribution $\Phi$ obtained in the third step S303 (evaluation of muscle aging (MA)). In this processing, the spatial mean of the phase angle distribution $\Phi$ may be calculated from the phase angle distribution $\Phi$ in the inside of the living body of the subject.

(Second processing S304B of fourth step)

In the second processing S304B of the fourth step, the local biological tissue fatty infiltration evaluation unit 54 is used to assign the phase angle distribution $\Phi$ obtained in the third step S303 to each local biological tissue of the subject, thereby creating the phase angle distribution $\Phi_p$ of the local biological tissue, which is the phase angle distribution of the local biological tissue (evaluation of local fatty infiltration (FI)). As a result, the local fatty infiltration (FI) can be evaluated. The spatial mean of the phase angle distribution $\Phi_p$ of the local biological tissue may be calculated from the phase angle distribution $\Phi_p$ of the local biological tissue.

(Third processing S304C of fourth step)

In the third processing S304C of the fourth step, the local biological tissue cross-sectional area evaluation unit 55 is used to perform binarization processing on the phase angle distribution of the local biological tissue, and then the local biological tissue cross-sectional area ratio is calculated (evaluation of local muscle cross-sectional area (CSA)). As a result, the local muscle cross-sectional area (CSA) can be evaluated.

The local biological tissue evaluation device 100 and the local biological tissue evaluation method according to the present embodiment have been described above. Since the local biological tissue evaluation device and the local biological tissue evaluation method according to the present embodiment evaluate the local biological tissue by bringing the electrode into contact with the skin of the subject, the local biological tissue evaluation device and the local biological tissue evaluation method are low-invasive, and are low-cost and simple as compared with the related art.

According to the local biological tissue evaluation device and the local biological tissue evaluation method according to the present embodiment, the phase angle distribution of the inside of the living body of the subject and the spatial mean of the phase angle distribution can be obtained. Therefore, for example, the muscle aging state can be quantitatively evaluated.

According to the local biological tissue evaluation device and the local biological tissue evaluation method according to the present embodiment, the spatial mean of the phase angle distribution of each local biological tissue can be obtained. Therefore, for example, the state of the local fatty infiltration of each muscle compartment can be quantitatively evaluated.

According to the local biological tissue evaluation device and the local biological tissue evaluation method according to the present embodiment, the local biological tissue cross-sectional area of the subject can be obtained. Therefore, for example, the local muscle cross-sectional area ratio of each muscle compartment can be quantitatively evaluated.

The local biological tissue evaluation device 100 includes the local biological tissue aging evaluation unit 53, the local biological tissue fatty infiltration evaluation unit 54, and the local biological tissue cross-sectional area evaluation unit 55, but may not include the local biological tissue fatty infiltration evaluation unit 54 and the local biological tissue cross-sectional area evaluation unit 55. In addition, the local biological tissue evaluation unit 60 need only have a function corresponding to the biological tissue.

In the local biological tissue evaluation method according to the present embodiment, the fourth step S304 includes the first processing S304A, the second processing S304B, and the third processing S304C, but may include any of these processes. For example, the second processing S304B and the third processing S304C may not be included.

(Examples)

Next, to verify the effectiveness of the local biological tissue evaluation device of the present disclosure, an example of an experiment using the local biological tissue evaluation device shown in FIG. 1 will be described, but the present invention is not limited to the following experimental examples.

(Experimental Example 1)

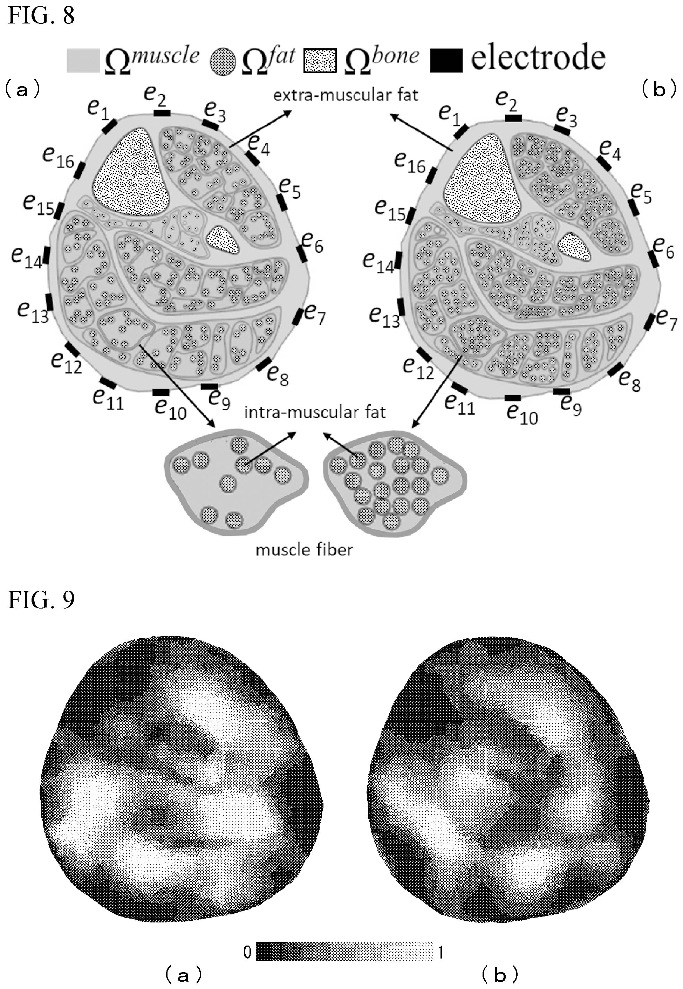

First, a simulation was performed. FIG. 8 is a configuration of a muscle used in the simulation of muscle quality evaluation. Here, the muscle was divided into four muscle compartments: a first muscle compartment (gastrocnemius muscle), a second muscle compartment (soleus muscle), a third muscle compartment (tibialis posterior muscle, flexor digitorum longus muscle, flexor hallucis longus muscle, and the like), and a fourth muscle compartment (anterior portion, extensor digitorum longus muscle, and fibularis longus muscle). (a) of FIG. 8 is a schematic cross-sectional view of a muscle having a high quality with a small amount of intramuscular fat. (b) of FIG. 8 is a schematic cross-sectional view of a muscle having a low quality with a large amount of intramuscular fat.

A simplified Maxwell's equation was used for the simulation. The equations used are Equations (20) and (21). In Equations (20) and (21), E [V/m] is an electric field, $\Phi$ [V] is an electric potential, and $\omega$ [rad/s] is an applied angular frequency and is $2\pi f$ (f [Hz] is an applied frequency). $\varepsilon$ [F/m] is a permittivity. Equations (22) to (25) were used as boundary conditions. In Equations (22) to (25), $\partial\Omega^{calf}$ is a boundary of the calf other than a cross section surrounded by an electrode. $I_0$[A] is an electrode injected current, and n is a boundary normal vector. In addition, $\partial\Omega^e$ is a boundary of the electrode. $\partial\Omega^{ei}$ and $\partial\Omega^{ej}$ are boundaries of an i-th electrode and a j-th electrode.

$$\nabla\cdot\mathbf{E}=0 \qquad (20)$$

$$\mathbf{E} = (\sigma + j\omega\varepsilon)\nabla\phi \qquad (21)$$

$$\mathbf{n}\cdot\mathbf{E} = 0 \qquad \text{on the } \partial\Omega^{calf} \qquad (22)$$

$$\int_{\partial\Omega^{e_i}} \mathbf{n}\cdot\mathbf{E}ds = I_0 \qquad \text{on the } \partial\Omega^{e_i} \qquad (23)$$

$$\int_{\partial\Omega^{e_j}} \mathbf{n}\cdot\mathbf{E}ds = -I_0 \qquad \text{on the } \partial\Omega^{e_j} \qquad (24)$$

$$\int_{\partial\Omega^e} \mathbf{n}\cdot\mathbf{E}\mathrm{d}s = 0 \qquad\qquad \text{on the } \partial\Omega^e \backslash\partial\Omega^{e_{i,j}} \tag{25}$$

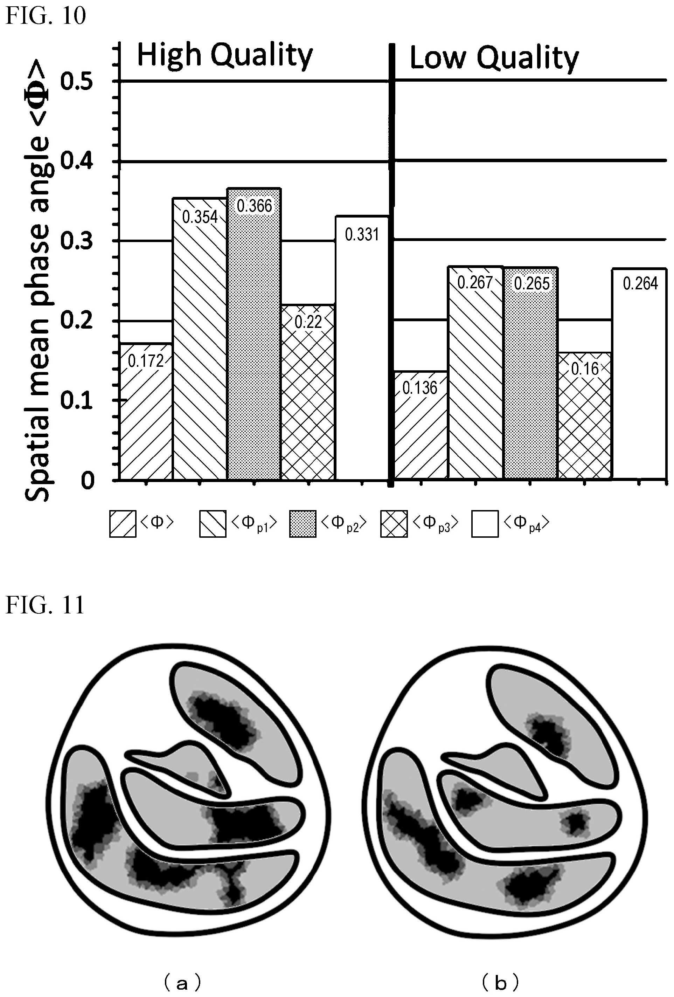

FIG. 9 is a diagram showing a calculated phase angle distribution image $\Phi$ using the local biological tissue evaluation device of the present disclosure. (a) of FIG. 9 is a phase angle distribution image in a case of a high-quality muscle calculated from the schematic cross-sectional view of (a) of FIG. 8, and (b) of FIG. 9 is a phase angle distribution image in a case of a low-quality muscle calculated from the schematic cross-sectional view of (b) of FIG. 8. In this diagram, a higher brightness indicates a position with a higher phase angle, that is, as shown in FIG. 9, it is possible to visualize where the position of the high-quality muscle is in each muscle compartment. Furthermore, to quantitatively evaluate the quality of the muscle, a spatial mean phase angle of each muscle compartment was determined. The obtained results are shown in FIG. 10. In FIG. 10, $<\Phi>$ is a spatial mean phase angle of the entire muscle, $<\Phi_{p1}>$ is a spatial mean phase angle of the first muscle compartment, $<\Phi_{p2}>$ is a spatial mean phase angle of the second muscle compartment, $<\Phi_{p3}>$ is a spatial mean phase angle of the third muscle compartment, and $<\Phi_{p4}>$ is a spatial mean phase angle of the fourth muscle compartment. In addition, High Quality in the figure is a case of the high-quality muscle shown in (a) of FIG. 8 and (a) of FIG. 9, and Low Quality is a case of the low-quality muscle shown in (a) of FIG. 8 and (a) of FIG. 9. In FIG. 10, the quality of the muscle can be quantitatively evaluated, that is, in $<\Phi>$, $<\Phi_{p1}>$, $<\Phi_{p2}>$, $<\Phi_{p3}>$, and $<\Phi_{p4}>$, it is possible to quantitatively know where the high-quality muscle is more or less in the muscle compartment.

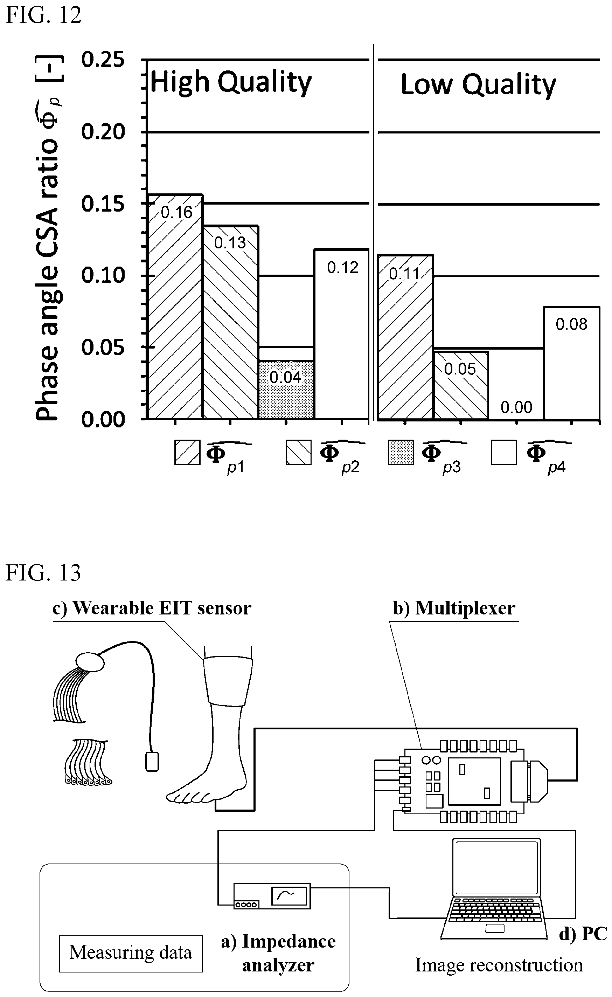

FIG. 11 shows a binarized phase angle distribution image $\Phi_p{}^{\wedge}$ calculated using the local biological tissue cross-sectional area evaluation unit of the present disclosure, in which a high phase angle is indicated in black. (a) of FIG. 11 is a phase angle distribution image in a case of a high-quality muscle, and (b) of FIG. 11 is a phase angle distribution image in a case of a low-quality muscle. In FIG. 11, it was confirmed that, in a case of the high-quality muscle, the black region (region with a high phase angle) was increased. To quantitatively evaluate, a local muscle cross-sectional area ratio (CSA ratio) between the muscle compartments was calculated using Equation (19). The obtained results are shown in FIG. 12. As shown in FIG. 12, in all of the first muscle compartment, the second muscle compartment, the third muscle compartment, and the fourth muscle compartment, the high-quality muscle showed a higher value of the CSA ratio.

(Experimental Example 2)

FIG. 13 shows a schematic diagram of a device used in Experimental Example 2. The present experimental device includes an impedance analyzer (HIOKI IM 3570) as a current-voltage measurement unit, a 6-channel multiplexer, an analog multiplexer/demultiplexer, a sensor (EIT sensor), and a personal computer as a calculation evaluation unit. Four ports (Lc, Lp, Hp, and Hc) of the impedance analyzer were expanded to 16 ports and used. The 16-channel multiplexer and 16 electrodes in the EIT sensor were connected using a coaxial cable. The complex impedance measured by the impedance analyzer was transmitted to the PC using USB communication.

The present experiment was performed on 14 healthy subjects (male, age of $30 \pm 7$ years, height of $173.5 \pm 6.5$ cm, BMI: $32.2 \pm 10.4$ kg/m$^2$) without musculoskeletal diseases or the like. There are two stages in the measurement protocol, the first stage is a stage in which the measurement is performed using the local biological tissue evaluation device of the present disclosure, and the second stage is a stage in which the measurement is performed using an ultrasound system (manufactured by GE Healthcare) for comparison. All measurements were performed in a state in which the subject was seated.

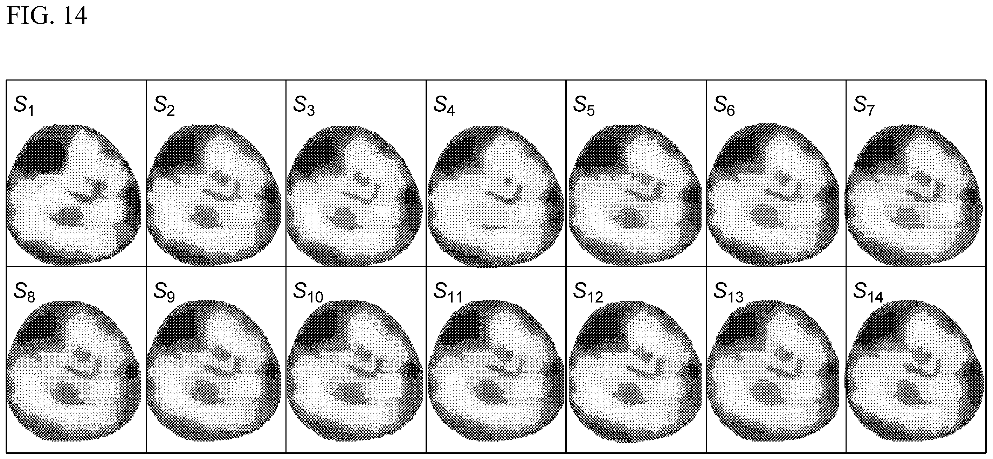

FIG. 14 is a phase angle distribution image $\Phi$ of 14 subjects calculated by the phase angle Jacobian matrix creation unit 51 and the local biological tissue aging evaluation unit 53, and the subjects are denoted by S1 to S14 in order from the subject with the youngest age. In this figure, the whiter the color is, the higher the phase angle is, and the blacker the color is, the lower the phase angle is, and it is possible to know which location of which muscle compartment has a high quality.

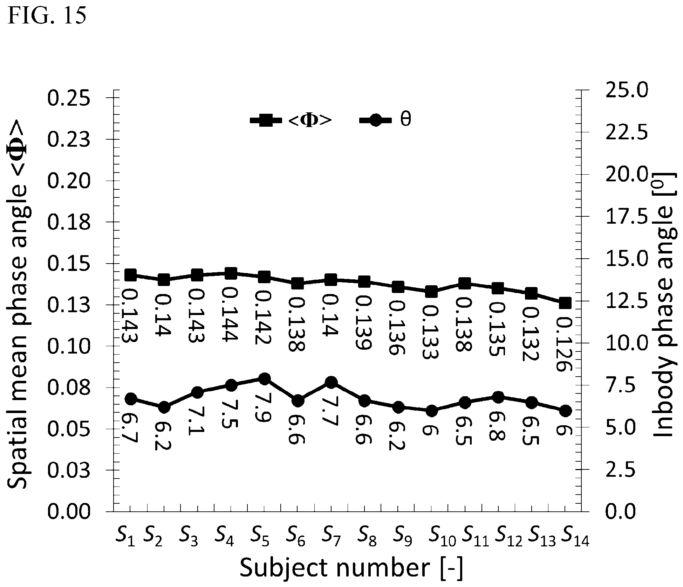

FIG. 15 shows the spatial mean phase angle $<\Phi>$ of each subject calculated from Equation (9), with the value indicated by a mark ■ and the y-axis on the left side. Although there is an individual difference, in general, there is a tendency that the value of $\Phi$ is higher in a subject with a lower age than in a subject with a higher age. For reference, the global phase angle obtained from a body composition analyzer (trade name of InBody S20) is shown by a mark ● and the value is shown on the y-axis on the right side, and although there is an individual difference, the same tendency is observed.

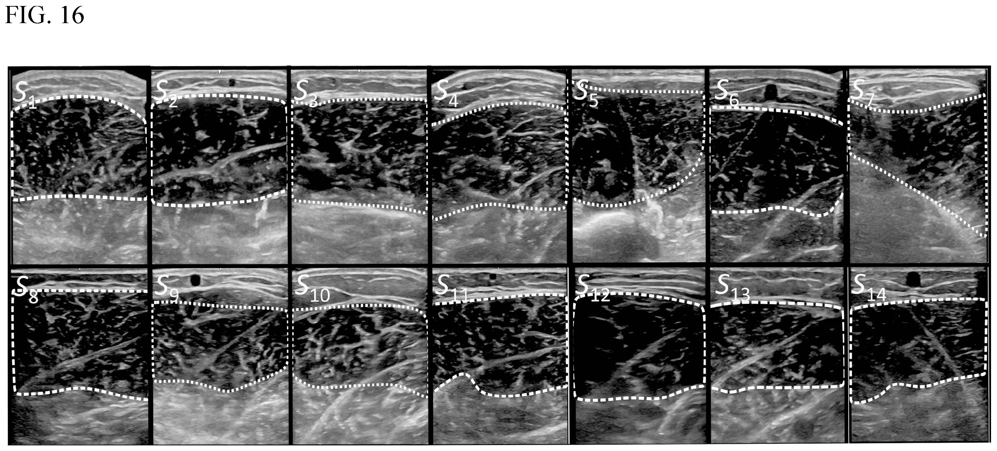

FIG. 16 is an ultrasound image of the calf of each subject, and a region surrounded by a broken line represents a part of the first muscle compartment (gastrocnemius muscle) in a representative manner, white to gray represents fat, and black represents muscle fibers. The local fatty infiltration uFI and the local muscle cross-sectional area uCSA were calculated using these ultrasound images (u indicates ultrasound). The uFI is defined as a ratio of the number of meshes indicating muscle fibers to the number of meshes occupied by fat in the ultrasound image. A low uFI value means that the proportion of fat is small and the quality of the muscle is high, and a high uFI value means that the proportion of fat is high and the quality of the muscle is low. The uCSA is the number of black muscle fiber meshes in the region surrounded by the broken line in the ultrasound image of FIG. 16 with respect to the number of meshes. A high uCSA value means that the proportion of muscle fibers is high and the quality of the muscle is high.

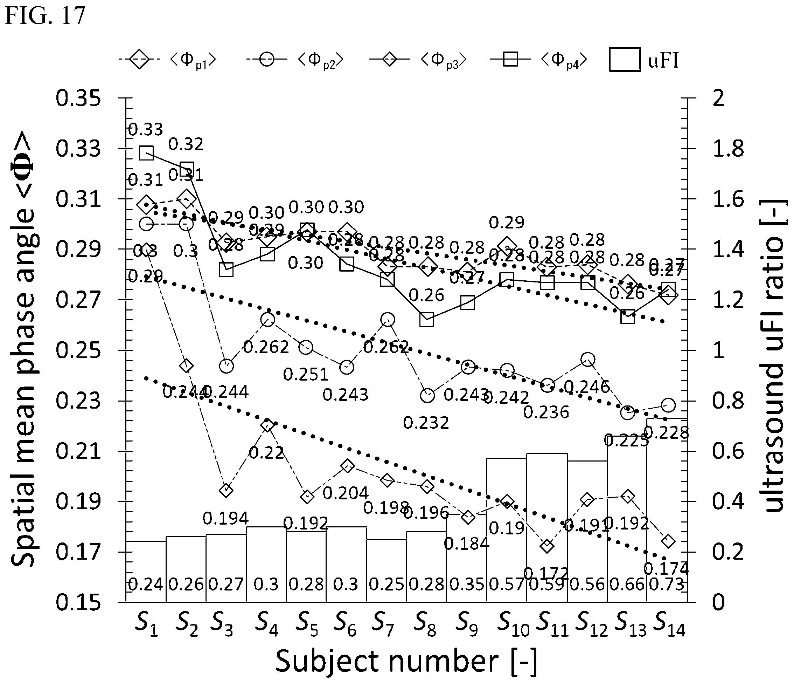

FIG. 17 shows a spatial mean phase angle $<\Phi_{p1}>$ of a first muscle compartment, a spatial mean phase angle $<\Phi_{p2}>$ of a second muscle compartment, a spatial mean phase angle $<\Phi_{p3}>$ of a third muscle compartment, a spatial mean phase angle $<\Phi_{p4}>$ of a fourth muscle compartment, and a local fatty infiltration uFI obtained from an ultrasound image of each subject. From this figure, it is possible to know which muscle compartment exhibits a high phase angle in each subject, and although there is an individual difference, there is a tendency that the phase angle decrease with an increase in age, which is reasonable in consideration of a general aging phenomenon. On the other hand, in the ultrasound image, the youngest subject has the small number of gray meshes, and the uFI increases with an increase in age, although there is an individual difference. These results qualitatively match the results of the phase angle image.

When looking at the details, the mean value of $<\Phi_{p1}>$ of 0.29 was the highest value in each muscle compartment. This is considered to be because the first muscle compartment has the largest muscle fibers that generate muscle strength. On the other hand, $<\Phi_{p3}>$ was the lowest value. It is presumed that the third compartment has small muscle fibers. The uFI increases with an increase in age, although there is an individual difference. This is considered to be because the fat in the muscle has increased. The tendency of the spatial mean phase angle $<\Phi_{p1}>$ of the first muscle compartment, the spatial mean phase angle $<\Phi_{p2}>$ of the second muscle compartment, the spatial mean phase angle $<\Phi_{p3}>$ of the third muscle compartment, and the spatial mean phase angle $<\Phi_{p4}>$ of the fourth muscle compartment is the same as the tendency of the local fatty infiltration uFI obtained from the ultrasound image, although there is an individual difference.

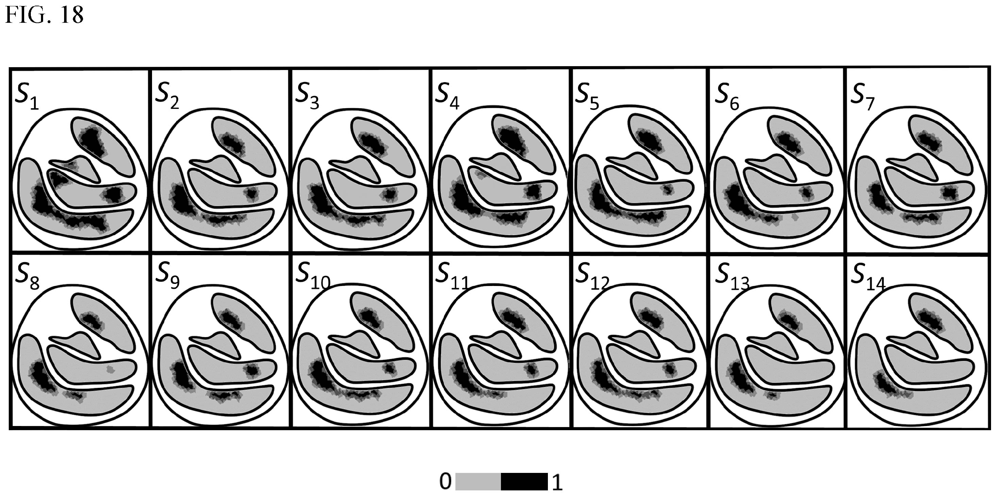

FIG. 18 shows the binarized phase angle image of each subject calculated by the local biological tissue cross-sectional area evaluation unit of the present disclosure. In this figure, gray indicates a position of a low phase angle, and black indicates a position of a high phase angle. As shown in FIG. 18, qualitatively, it can be seen that the subject with the youngest age has the widest black mesh region.

[Table 1]

| Items | Young (Age $\leq$ 30) | Old (Age > 30) |
|---|---|---|
| Mean | 0.1414 | 0.1341 |
| SD | 0.0021 | 0.0044 |
| SE | 0.0008 | 0.0017 |
| Interval | 0.1434 | 0.1382 |
| Mean Diff. | 0.0073 | |
| $t_{0.025}(12)$ | 2.1790 | |
| $t$-value | 3.9544 | |
| $p$-value | < 0.001 | |

Among the three muscle quality indices, the local muscle aging (MA) will be focused on and discussed.

Table 1 shows the results of the independent t-test for the spatial mean phase angle $<\Phi>$ between the younger subject group and the older subject group. The null hypothesis is that the values of $<\Phi>$ of the younger subject group and the older subject group are equal. Since the t-value = 3.9544 > 0 is greater than t0.025 (12) = 2.1790, the null hypothesis is rejected, and as a result, it can be concluded that $<\Phi>$ is significantly related to the local muscle aging (n = 14, p < 0.001) of the younger subject group (age $\leq$ 30) and the older subject group (age > 30). Further, it can be seen that $<\Phi>$ of the younger subject group is significantly higher than that of the older subject group.

[Table 2]

| Items | $<\Phi>$ | | | | | | | | uFI | |
| | $<\Phi_{p1}>$ | | $<\Phi_{p2}>$ | | $<\Phi_{p3}>$ | | $<\Phi_{p4}>$ | | | |
| | Young | Old | Young | Old | Young | Old | Young | Old | Young | Old |
|---|---|---|---|---|---|---|---|---|---|---|
| Mean | 0.3894 | 0.3674 | 0.1776 | 0.1531 | 0.8466 | 0.7514 | 0.1576 | 0.1451 | 0.2714 | 0.5343 |
| SD | 0.0142 | 0.0111 | 0.015 | 0.0135 | 0.0823 | 0.033 | 0.0073 | 0.0055 | 0.0234 | 0.1622 |
| SE | 0.0054 | 0.0042 | 0.0057 | 0.0051 | 0.0311 | 0.0125 | 0.0027 | 0.0021 | 0.0088 | 0.0613 |
| Interval | 0.4026 | 0.3777 | 0.1915 | 0.1656 | 0.9227 | 0.7819 | 0.1643 | 0.1502 | 0.2931 | 0.6843 |
| Mean Diff. | 0.022 | | 0.0244 | | 0.0951 | | 0.0124 | | -0. 2629 | |
| $t_{0.025}(12)$ | 2.1790 | | | | | | | | -2.790 | |

(continued)

| Items | <Φ> | | | | | | | | uFI | |
|---|---|---|---|---|---|---|---|---|---|---|
| | $\langle\Phi_{p1}\rangle$ | | $\langle\Phi_{p2}\rangle$ | | $\langle\Phi_{p3}\rangle$ | | $\langle\Phi_{p4}\rangle$ | | | |
| | Young | Old | Young | Old | Young | Old | Young | Old | Young | Old |
| *t*-value | 3.2358 | | 3.202 | | 2.8393 | | 3.6219 | | -4.2448 | |
| *p*-value | 0.0036 | | 0.0038 | | 0.0075 | | 0.0018 | | 0.0006 | |

Among the three muscle quality indices, the local fatty infiltration (FI) will be focused on and discussed.

Table 2 shows the results of the independent t-test for the spatial mean phase angle $\langle\Phi_p\rangle$ of each muscle compartment and the local fatty infiltration uFI by ultrasound between the younger subject group and the older subject group. The null hypothesis is that the values of $\langle\Phi_p\rangle$ of the younger subject group and the older subject group are equal. The t-value of each muscle compartment ($\langle\Phi_{p1}\rangle = 3.4412 > 0$, $\langle\Phi_{p2}\rangle = 2.2478 > 0$, $\langle\Phi_{p3}\rangle = 2.9055 > 0$, $\langle\Phi_{p4}\rangle = 3.2504 > 0$) was greater than t0.025 (12) = 2.1790. Furthermore, the null hypothesis that the values of uFI of the younger subject group and the older subject group are equal is rejected because the t-value of uFI of -4.2448 < 0 is less than t0.025 (12) = 2.1790. From the above results, it can be concluded that $\langle\Phi_p\rangle$ and uFI are significantly related to the local muscle aging (n = 14, p < 0.05) of the younger subject group (age ≤ 30) and the older subject group (age > 30). Furthermore, $\langle\Phi\rangle$ of the younger subject group is significantly higher than that of the older subject group. In contrast, uFI of the younger subject group is significantly lower than that of the older subject group.

[Table 3]

| Items | $\widehat{\Phi_p}$ | | | | | | | | uCSA | |
|---|---|---|---|---|---|---|---|---|---|---|
| | $\widehat{\Phi_{p1}}$ | | $\widehat{\Phi_{p2}}$ | | $\widehat{\Phi_{p3}}$ | | $\widehat{\Phi_{p4}}$ | | | |
| | Young | Old | Young | Old | Young | Old | Young | Old | Young | Old |
| Mean | 0.0486 | 0.0271 | 0.27 | 0.2586 | 0.0286 | 0.0129 | 0.1586 | 0.1457 | 0.5184 | 0.4833 |
| SD | 0.0135 | 0.0095 | 0.01 | 0.009 | 0.0122 | 0.0076 | 0.0069 | 0.0079 | 0.0149 | 0.0294 |
| SE | 0.0051 | 0.0036 | 0.0038 | 0.0034 | 0.0046 | 0.0029 | 0.0026 | 0.003 | 0.0056 | 0.0111 |
| Interval | 0.061 | 0.0359 | 0.2792 | 0.2669 | 0.0398 | 0.0198 | 0.165 | 0.153 | 0.5322 | 0.5105 |
| Mean Diff. | 0.0214 | | 0.0114 | | 0.0157 | | 0.0129 | | 0.0351 | |
| *t*-value | 3.4412 | | 2.2478 | | 2.9055 | | 3.2504 | | 2.8216 | |
| *p*-value | 0.0024 | | 0.00221 | | 0.0066 | | 0.0035 | | 0.0077 | |

Among the three muscle quality indices, the local muscle cross-sectional area (CSA) will be focused on and discussed. The subjects are divided into two groups, a younger subject group and an older subject group, and an independent t-test is performed between the two groups for the local muscle cross-sectional area $CSA_{\varphi M}$ obtained by the local biological tissue evaluation method according to the embodiment of the present invention and the ultrasound muscle region uCSA. Table 3 shows the results of the independent t-test. The null hypothesis is that the values of $\varphi_p$ of the younger subject group and the older subject group are equal. The t-value of each muscle compartment ($\varphi_{p1} = 3.4412 > 0$, $\varphi_{p2} = 2.2478 > 0$, $\varphi_{p3} = 2.9055 > 0$, $\varphi_{p4} = 3.2504 > 0$) was greater than t0.025 (12) = 2.1790. In addition, the t-value of uCSA of 2.8216 > 0 is greater than t0.025 (12) = 2.1790. Therefore, the null hypothesis that the values of uCSA of the younger subject group and the older subject group are equal is rejected. It can be concluded that $\varphi_p$ and uCSA are significantly related to the local muscle aging (n = 14, p < 0.05) of the younger subject group (age ≤ 30) and the older subject group (age > 30). Furthermore, $\varphi_p$ and uCSA of the younger subject group are significantly higher than those of the older subject group.

From the above results, it is confirmed that the age of the subject group and the muscle aging (MA), the local fatty infiltration (FI), and the local muscle cross-sectional area (CSA) have a high correlation. In general, the younger the age is, the higher the quality of the local biological tissue is, and there is a high correlation with the results of the ultrasound image. Therefore, it is presumed that the quality of the local biological tissue in the muscle compartment or the like can be evaluated by using the local biological tissue evaluation device according to the present disclosure.

REFERENCE SIGNS LIST

10 Phase angle variation amount measurement unit
50 Calculation evaluation unit
51 Phase angle Jacobian matrix creation unit
52 Image reconstruction unit
53 Local biological tissue aging evaluation unit
54 Local biological tissue fatty infiltration evaluation unit
55 Local biological tissue cross-sectional area evaluation unit
56 Output unit

**Claims**

1. A local biological tissue evaluation device comprising:

   a phase angle variation amount measurement unit including a sensor having a plurality of electrodes placeable on skin of a subject spaced apart from each other, and configured to inject a current or a voltage between ones of the electrodes in a state in which each of the electrodes is in contact with the skin, to measure a voltage based on a current-injection voltage-measurement pattern when the current is injected, to measure a current based on a voltage-injection current-measurement pattern when the voltage is injected between the electrodes, and to measure a feature amount obtained based on a variation amount of a phase angle for the current-injection voltage-measurement pattern or the voltage-injection current-measurement pattern;
   a phase angle Jacobian matrix creation unit configured to create a phase angle Jacobian matrix based on the current-injection voltage-measurement pattern or the voltage-injection current-measurement pattern; and
   an image reconstruction unit configured to image a phase angle distribution inside of a living body of the subject using the feature amount and the phase angle Jacobian matrix.

2. The local biological tissue evaluation device according to Claim 1,
   wherein the phase angle Jacobian matrix creation unit creates mesh coordinates of a measurement location of the subject and an equation of a relationship of an n-th mesh in an m-th measurement pattern, and derives, as an element of the phase angle Jacobian matrix obtained as a solution of the equation, a current and an electric potential at each mesh coordinate, and a phase angle of an impedance, which is a ratio of the voltage to the current.

3. The local biological tissue evaluation device according to Claim 1 or 2,

   wherein the feature amount is a phase angle difference represented by Equation (1),

   $$\Delta\theta_m = \theta_m(f) - \theta_m(f_0) \dots \text{Equation (1)}$$

   in Equation (1),
   $\Delta\theta_m$ is a phase angle difference in an m-th current-injection voltage-measurement pattern or in an m-th voltage-injection current-measurement pattern,
   $\theta_m(f)$ is a phase angle at a high frequency f in the m-th current-injection voltage-measurement pattern or in the m-th voltage-injection current-measurement pattern, and
   $\theta_m(f_0)$ is a phase angle at a low frequency $f_0$ in the m-th current-injection voltage-measurement pattern or in the m-th voltage-injection current-measurement pattern.

4. The local biological tissue evaluation device according to Claim 1 or 2, further comprising:
   a local biological tissue evaluation unit configured to evaluate a local biological tissue using the imaged phase angle distribution.

5. The local biological tissue evaluation device according to Claim 4,
   wherein the local biological tissue evaluation unit includes a local biological tissue aging evaluation unit configured to calculate a spatial mean of the phase angle distribution from the phase angle distribution.

6. The local biological tissue evaluation device according to Claim 4,
   wherein the local biological tissue evaluation unit further includes a local biological tissue fatty infiltration evaluation unit configured to create a local biological tissue phase angle distribution, which is a phase angle distribution of a local biological tissue, by assigning the phase angle distribution to each local biological tissue of the subject.

7.  The local biological tissue evaluation device according to Claim 6,
    wherein the local biological tissue fatty infiltration evaluation unit calculates a spatial mean of the local biological tissue phase angle distribution from the local biological tissue phase angle distribution.

8.  The local biological tissue evaluation device according to Claim 6,
    wherein the local biological tissue evaluation unit further includes a local biological tissue cross-sectional area evaluation unit configured to calculate a local biological tissue cross-sectional area ratio after performing binarization processing on the local biological tissue phase angle distribution.

9.  A local biological tissue evaluation method comprising:

    a first step of, using a sensor including a plurality of electrodes placeable on skin of a subject spaced apart from each other, injecting a current or a voltage between ones of the electrodes in a state in which each of the electrodes is in contact with the skin, measuring a voltage based on a current-injection voltage-measurement pattern when the current is injected, measuring a current based on a voltage-injection current-measurement pattern when the voltage is injected between the electrodes, and measuring a feature amount obtained based on a variation amount of a phase angle for the current-injection voltage-measurement pattern or the voltage-injection current-measurement pattern;
    a second step of creating a phase angle Jacobian matrix based on the current-injection voltage-measurement pattern or the voltage-injection current-measurement pattern; and
    a third step of imaging a phase angle distribution inside of a living body of the subject from the feature amount and the phase angle Jacobian matrix.

10. The local biological tissue evaluation method according to Claim 9,
    wherein in the second step, mesh coordinates of a measurement location of the subject and an equation of a relationship of an n-th mesh in an m-th measurement pattern are created, and a current and an electric potential at each mesh coordinate, and a phase angle of an impedance, which is a ratio of the voltage to the current, are derived as an element of the phase angle Jacobian matrix obtained as a solution of the equation.

11. The local biological tissue evaluation method according to Claim 9 or 10,

    wherein the feature amount is a phase angle difference represented by Equation (2),

$$\Delta\theta_m = \theta_m(f) - \theta_m(f_0) \ ... \ \text{Equation (2)}$$

    in Equation (2),
    $\Delta\theta_m$ is a phase angle difference in an m-th current-injection voltage-measurement pattern or in an m-th voltage-injection current-measurement pattern,
    $\theta_m(f)$ is a phase angle of a high frequency f in the m-th current-injection voltage-measurement pattern or in the m-th voltage-injection current-measurement pattern, and
    $\theta_m(f_0)$ is a phase angle of a low frequency $f_0$ in the m-th current-injection voltage-measurement pattern or in the m-th voltage-injection current-measurement pattern.

12. The local biological tissue evaluation method according to Claim 9 or 10, further comprising:
    a fourth step of evaluating a local biological tissue using the imaged phase angle distribution.

13. The local biological tissue evaluation method according to Claim 12,
    wherein in the fourth step, a spatial mean of the phase angle distribution is calculated from the phase angle distribution.

14. The local biological tissue evaluation method according to Claim 12,
    wherein in the fourth step, the phase angle distribution is assigned to each local biological tissue of the subject to create a local biological tissue phase angle distribution, which is a phase angle distribution of the local biological tissue.

15. The local biological tissue evaluation method according to Claim 14,
wherein in the fourth step, after binarization processing is performed on the local biological tissue phase angle distribution, a local biological tissue cross-sectional area ratio is calculated.

FIG. 1

FIG. 2

FIG. 3

S101

APPLIED CURRENT PATTERN ($1 \leqq m \leqq M$) SETTING

S102

APPLIED CURRENT $I_m(f_0)$
OF LOW FREQUENCY f0,
MEASURED VOLTAGE $V_m(f_0)$
OF LOW FREQUENCY f0

S103

APPLIED CURRENT $I_m(f)$
OF HIGH FREQUENCY f,
MEASURED VOLTAGE $V_m(f)$
OF HIGH FREQUENCY f

S104

RESISTANCE $Z'_m(f_0)$ OF LOW FREQUENCY f0,
REACTANCE $Z''_m(f_0)$ OF LOW FREQUENCY f0

S105

RESISTANCE $Z'_m(f)$ OF HIGH FREQUENCY f,
REACTANCE $Z''_m(f)$ OF HIGH FREQUENCY f

PHASE ANGLE CALCULATION EQUATION $\theta_m = \tan^{-1}(Z''_m / Z'_m)$  S106

S107

MEASURED PHASE ANGLE $\theta_m(f_0)$
OF LOW FREQUENCY f0

S108

MEASURED PHASE ANGLE $\theta_m(f)$
OF HIGH FREQUENCY f

MEASURED PHASE ANGLE DIFFERENCE
$\Delta\theta_m = \theta_m(f) - \theta_m(f_0)$  S109

FIG. 4

START

CREATION OF MESH COORDINATES OBTAINED BY
PARTITIONING BASED ON CONTOUR $\partial\Omega$ OF SUBJECT
MEASUREMENT LOCATION AND INSIDE OF LIVING BODY $\Omega$ ~S201

DETERMINATION OF CURRENT-APPLICATION
VOLTAGE-MEASUREMENT PATTERN OR
VOLTAGE-APPLICATION CURRENT-MEASUREMENT PATTERN ~S202

~S203
CREATION OF RESISTANCE
JACOBIAN MATRIX $^{Z'}J$

~S204
CREATION OF REACTANCE
JACOBIAN MATRIX $^{Z''}J$

CREATION OF PHASE ANGLE JACOBIAN MATRIX $^{\Phi}J$ ~S205

END

FIG. 5

FIG. 6

IMAGE OF PHASE ANGLE DISTRIBUTION
$\Phi_p$ IN P-TH SECTION
*WHITER INDICATES HIGHER VALUES
IN THIS FIGURE

IMAGE OF BINARIZED PHASE
ANGLE DISTRIBUTION $\widehat{\Phi_p}$
*BLACKER INDICATES HIGHER
VALUES IN THIS FIGURE

FIG. 7

START

S301
MEASUREMENT OF
PHASE ANGLE $\theta$

S302
CREATION OF PHASE ANGLE
JACOBIAN MATRIX $^\Phi J$

S303
IMAGING OF PHASE
ANGLE DISTRIBUTION $\Phi$

S304

S304A
EVALUATION OF
MUSCLE AGING (MA)

S304B
EVALUATION OF
LOCAL FATTY
INFILTRATION (FI)

S304C
EVALUATION OF LOCAL
MUSCLE CROSS-SECTIONAL
AREA (CSA)

END

FIG. 8

FIG. 9

FIG. 10

FIG. 11

（a）                    （b）

## FIG. 12

## FIG. 13

FIG. 14

FIG. 15

FIG. 16

EP 4 778 455 A1

FIG.17

FIG. 18

# EP 4 778 455 A1

## INTERNATIONAL SEARCH REPORT

International application No.

**PCT/JP2024/032539**

### A. CLASSIFICATION OF SUBJECT MATTER

***A61B 5/0536***(2021.01)i

FI: A61B5/0536

According to International Patent Classification (IPC) or to both national classification and IPC

### B. FIELDS SEARCHED

Minimum documentation searched (classification system followed by classification symbols)

A61B5/05

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2021-97916 A (UNIV CHIBA NAT UNIV CORP.) 01 July 2021 (2021-07-01) paragraphs [0020]-[0060], fig. 1-16 | 1, 4-5, 9, 12-13 |
| A | | 2-3, 6-8, 10-11, 14-15 |
| Y | YASUNORI, Akamatsu et al. Phase angle from bioelectrical impedance analysis is a useful indicator of muscle quality. Journal of Cachexia, Sarcopenia and Muscle. 2022, 13, 180-189 abstract, p.1 | 1, 4-5, 9, 12-13 |
| Y | GUNES, Cagdas et al. A Comparison Between Electrical Capacitance Tomography and Displacement-Current Phase Tomography. IEEE SENSORS JOURNAL. 2017, vol. 17, no. 24, 8037-8046 II. ECT AND DCPT, pp. 8038-8039 | 1, 4-5, 9, 12-13 |

☐ Further documents are listed in the continuation of Box C.    ☑ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **25 September 2024** | **03 December 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/032539**

| Patent document cited in search report | Publication date (day/month/year) | Patent family member(s) | Publication date (day/month/year) |
|---|---|---|---|
| JP 2021-97916 A | 01 July 2021 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023149511 A

**Non-patent literature cited in the description**

- **Y. JIANG** ; **M. SOLEIMANI**. Capacitively Coupled Phase-based Dielectric Spectroscopy Tomography. *Sci. Rep.*, 2018, vol. 8 (1), 17526